# EUROPÄISCHE PATENTSCHRIFT

(11) **EP 0 789 025 B1**
(45) Veröffentlichungstag und Bekanntmachung des Hinweises auf die Patenterteilung: **04.04.2001**
(21) Anmeldenummer: 97101509.4
(22) Anmeldetag: 31.01.1997
(51) Int. Cl.: C07D 413/14, C07D 417/14, C07D 413/04, C07D 417/04, C07D 263/58, A61K 31/42, A61K 31/425, A61K 31/44, A61K 31/495

(54) **Substituierte Oxazolidinone und ihre Verwendung als antibakterielle Arzneimittel**
Substituted oxazolidinone and their use as antibacterial medicine
Oxazolidinones substituées et leur utilisation comme médicaments antibactériens

(30) Priorität: 06.02.1996 DE 19604223
(43) Veröffentlichungstag der Anmeldung: 13.08.1997
(73) Patentinhaber: BAYER AG, 51368 Leverkusen (DE)
(72) Erfinder: Riedl, Bernd, Dr., 42115 Wuppertal (DE); Häbich, Dieter, Dr., 42115 Wuppertal (DE); Stolle, Andreas, Dr., 42115 Wuppertal (DE); Ruppelt, Martin, Dr., 42329 Wuppertal (DE); Bartel, Stephan, Dr., 51465 Bergisch Gladbach (DE); Guarnieri, Walter, Dr., 53909 Zülpich (DE); Endermann, Rainer, Dr., 42113 Wuppertal (DE); Kroll, Hein-Peter, Dr., 42115 Wuppertal (DE)

(56) Entgegenhaltungen:
- EP-A- 0 352 781
- EP-A- 0 693 491
- EP-A- 0 694 543
- DE-A- 4 425 609
- US-A- 4 801 600
- JOURNAL OF MEDICINAL CHEMISTRY, Bd. 35, Nr. 6, 20.März 1992, Seiten 1156-1165, XP000567006 CHUNG-HO PARK ET AL: "Antibacterials.Synthesis and structure-activity studies of 3-aryl-2-oxooxazolidines.4.Multiply-substi tuted aryl derivatives."

## Beschreibung

Die vorliegende Erfindung betrifft neue substituierte Oxazolidinone, Verfahren zu ihrer Herstellung und ihre Verwendung als Arzneimittel, insbesondere als antibakterielle Arzneimittel.

Aus den Publikationen US 5 254 577, US 4 705 799, EP 311 090, EP 312 000 und C.H. Park et al., J. Med. Chem. 35, 1156 (1992) sind N-Aryloxazolidinone mit antibakterieller Wirkung bekannt. Außerdem sind 3-(Stickstoff-substituierte)phenyl-5-beta -amidomethyloxazolidin-2-one aus der EP 609 905 A1 bekannt.

Ferner sind in der EP 609 491 und EP 657 440 Oxazolidinonderivate mit einer Monoaminoxidase inhibitorischen Wirkung und in der EP 645 376 mit Wirkung als Adhäsionsrezeptor-Antagonisten publiziert.

Antibakteriell wirksame Oxazolidinonderivate sind auch in unseren Anmeldungen EP 694 543, EP 693 491, EP 694 544, EP 697 412 und EP 738 726 beschrieben.

Die vorliegende Erfindung betrifft neue substituierte Oxazolidinone der allgemeinen Formel (I) 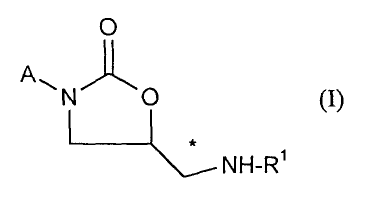 in welcher
- A: für einen über ein Kohlenstoffatom direkt gebundenen 5-gliedrigen aromatischen Heterocyclus mit bis zu 3 Heteroatomen aus der Reihe S, N und/oder O steht, der zusätzlich einen annellierten Benzol- oder Naphthylring besitzen kann, oder
für einen über ein Kohlenstoffatom direkt gebundenen 6-gliedrigen, aromatischen Heterocyclus mit mindestens einem Stickstoffatom steht, oder
für einen über ein Kohlenstoffatom direkt gebundenen, jeweils 6-gliedrigen, bi- oder tricyclischen aromatischen Rest mit mindestens einem stickstoffhaltigen Ring steht, oder
für β-Carbolin-3-yl oder für über den 6-Ring direkt gebundenes Indolizinyl steht, wobei die Cyclen gegebenenfalls jeweils bis zu 3-fach gleich oder verschieden durch Carboxy, Halogen, Cyano, Mercapto, Formyl, Phenyl, Trifluormethyl, Nitro, geradkettiges oder verzweigtes Alkoxy, Alkoxycarbonyl, Alkylthio oder Acyl mit jeweils bis zu 6 Kohlenstoffatomen oder durch geradkettiges oder verzweigtes Alkyl oder Alkenyl mit jeweils bis zu 6 Kohlenstoffatomen substituiert sind, die ihrerseits durch Phenyl substituiert sein können, und/oder durch Pyridyl substituiert sind, das seinerseits durch geradkettiges Alkyl oder Alkoxy mit jeweils bis zu 6 Kohlenstoffatomen substituiert sein kann oder
für einen Rest der Formel 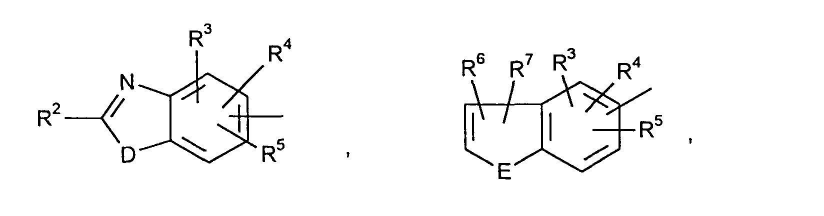 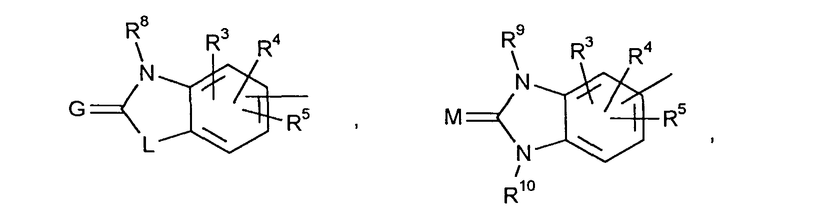 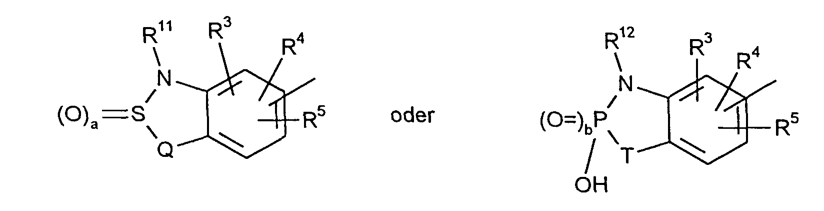 steht,
worin
R³, R⁴, R⁵, R⁶ und R⁷ gleich oder verschieden sind und Wasserstoff oder Carboxy, Halogen, Cyano, Formyl, Trifluormethyl, Nitro, geradkettiges oder verzweigtes Alkyl mit bis zu 6 Kohlenstoffatomen oder eine Gruppe der Formel -CO-NR¹³R¹⁴ bedeuten,
worin
R¹³ und R¹⁴ gleich oder verschieden sind und Wasserstoff, geradkettiges oder verzweigtes Alkyl mit bis zu 4 Kohlenstoffatomen oder Phenyl bedeuten,
R², R⁸, R⁹, R¹⁰, R¹¹ und R¹² gleich oder verschieden sind und Wasserstoff, Cycloalkylcarbonyl oder Cycloalkyl mit jeweils 3 bis 6 Kohlenstoffatomen, oder geradkettiges oder verzweigtes Alkoxycarbonyl oder Alkylthio mit jeweils bis zu 6 Kohlenstoffatomen bedeuten, oder geradkettiges oder verzweigtes Alkyl mit bis zu 10 Kohlenstoffatomen bedeuten, das gegebenenfalls durch Cyano, Trifluormethyl, Halogen, Phenyl, Hydroxy, Carboxyl, geradkettiges oder verzweigtes Alkoxycarbonyl mit bis zu 6 Kohlenstoffatomen, Aryl mit 6 bis 10 Kohlenstoffatomen, Cycloalkyl mit 3 bis 6 Kohlenstoffatomen und/oder durch eine Gruppe der Formel -(CO)_{c}-NR¹⁵R¹⁶, R¹⁷-N-SO₂-R¹⁸, R¹⁹R²⁰-N-SO₂- oder R²¹-S(O)_{d} substituiert ist,
worin
c eine Zahl 0 oder 1 bedeutet,
R¹⁵, R¹⁶ und R¹⁷ die oben angegebene Bedeutung von R¹³ und R¹⁴ haben und mit dieser gleich oder verschieden sind, oder gemeinsam mit dem Stickstoffatom einen 5- bis 6-gliedrigen, gesättigten Heterocyclus mit gegebenenfalls einem weiteren Heteroatom aus der Serie N, S und/oder O bilden, der seinerseits gegebenenfalls, auch an einem weiteren Stickstoffatom, durch geradkettiges oder verzweigtes Alkyl oder Acyl mit bis zu 3 Kohlenstoffatomen substituiert sein kann,
R¹⁸ und R²¹ gleich oder verschieden sind und geradkettiges oder verzweigtes Alkyl mit bis zu 4 Kohlenstoffatomen, Benzyl, Phenyl oder Tolyl bedeuten, oder
geradkettiges oder verzweigtes Acyl mit bis zu 6 Kohlenstoffatomen bedeuten, das gegebenenfalls durch Trifluormethyl, Trichlormethyl oder durch eine Gruppe der Formel -OR²² substituiert ist,
worin
R²² Wasserstoff oder geradkettiges oder verzweigtes Alkyl mit bis zu 6 Kohlenstoffatomen bedeutet, das gegebenenfalls durch Aryl mit bis zu 10 Kohlenstoffatomen substituiert ist, oder
eine Gruppe der Formel -(CO)ₑ-NR²³R²⁴, -NR²⁵-SO₂R²⁶, R²⁷R²⁸-NSO₂- oder R²⁹-S(O)_{f} bedeuten,
worin
e die oben angegebene Bedeutung von c hat und mit dieser gleich oder verschieden ist,
R²³ und R²⁴ und R²⁵ jeweils die oben angegebene Bedeutung von R¹⁵, R¹⁶ und R¹⁷ haben und mit dieser gleich oder verschieden sind,
R²⁷ und R²⁸ die oben angegebene Bedeutung von R¹³ und R¹⁴ haben und mit dieser gleich oder verschieden sind,
f die oben angegebene Bedeutung von d hat und mit dieser gleich oder verschieden ist,
R²⁶ und R²⁹ die jeweils oben angegebene Bedeutung von R¹⁸ und R²¹ haben und mit dieser gleich oder verschieden sind,
D ein Sauerstoffatom oder einen Rest der Formel -S(O)_{g} bedeutet,
worin
g eine Zahl 0, 1 oder 2 bedeutet,
E und L gleich oder verschieden sind und ein Sauerstoff- oder ein Schwefelatom bedeuten,
G, M, T und Q gleich oder verschieden sind und ein Sauerstoff- oder ein Schwefelatom, oder eine Gruppe der Formel -NR³⁰ bedeuten,
worin
R³⁰ Wasserstoff oder geradkettiges oder verzweigtes Alkyl mit bis zu 5 Kohlenstoffatomen bedeutet,
a und b gleich oder verschieden sind und eine Zahl 1 oder 2 bedeuten,
- R¹: für einen Rest der Formel 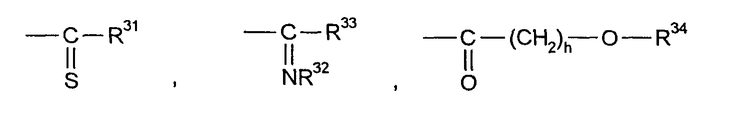 oder 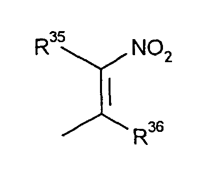 steht,
worin
R³¹ geradkettiges oder verzweigtes Alkyl mit bis zu 7 Kohlenstoffatomen, Cycloalkyl mit 3 bis 6 Kohlenstoffatomen, Phenyl oder eine Gruppe der Formel -NR³⁸R³⁹ bedeutet,
worin
R³⁸ und R³⁹ die oben angegebene Bedeutung von R¹³ und R¹⁴ haben und mit dieser gleich oder verschieden sind,
R³² Wasserstoff, Cyano, Cycloalkyl mit 3 bis 6 Kohlenstoffatomen, Phenyl oder geradkettiges oder verzweigtes Alkyl mit bis zu 7 Kohlenstoffatomen bedeutet,
R³³ Wasserstoff geradkettiges oder verzweigtes Alkyl mit bis zu 7 Kohlenstoffatomen, Phenyl, Cycloalkyl mit 3 bis 6 Kohlenstoffatomen oder eine Gruppe der Formel -NR⁴⁰R⁴¹ bedeutet,
worin
R⁴⁰ und R⁴¹ die oben angegebene Bedeutung von R¹³ und R¹⁴ haben und mit dieser gleich oder verschieden sind,
h eine Zahl 1, 2, 3 oder 4 bedeutet,
R³⁴ Wasserstoff, geradkettiges oder verzweigtes Alkyl mit bis zu 6 Kohlenstoffatomen oder Benzyl bedeutet,
R³⁵ und R³⁶ gleich oder verschieden sind und Wassserstoff oder geradkettiges oder verzweigtes Alkyl mit bis zu 6 Kohlenstoffatomen bedeuten, oder
- R¹: für Cyano oder für einen 5- bis 7-gliedrigen, gesättigten, partiell ungesättigten oder ungesättigten Heterocyclus mit bis zu 3 Heteroatomen aus der Reihe S, N und/oder O steht, der gegebenenfalls auch über eine N-Funktion, bis zu 2-fach gleich oder verschieden durch Benzyl, Halogen oder durch geradkettiges oder verzweigtes Alkyl mit bis zu 5 Kohlenstoffatomen substituiert ist,
und deren Salze.

Physiologisch unbedenkliche Salze der substituierten Oxazolidinone können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Als Salze können Salze mit üblichen Basen genannt werden, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldiisopropylamin, Prokain, Dibenzylamin, N-Methylmorpholin, Dihydroabietylamin, 1-Ephenamin oder Methyl-piperidin.

Als Salze können außerdem Reaktionsprodukte mit C₁-C₄-Alkylhalogeniden, insbesondere mit C₁-C₄-Alkyljodiden fungieren.

Heterocyclus steht im allgemeinen für einen 5- bis 6-gliedrigen, gesättigten oder ungesättigten Ring, der als Heteroatome bis zu 3 Sauerstoff-, Schwefel- und/oder Stickstoffatome enthalten kann. Bevorzugt werden genannt: Thienyl, Furyl, Pyrrolyl, Pyrazolyl, Pyridyl, Pyrimidyl, Pyrazinyl, Pyridazinyl, Thiazolyl, Oxazolyl, Imidazolyl, Pyrrolidinyl, Piperidinyl oder Piperazinyl.

Dazu gehören auch über N-gebundene, 5- bis 6-gliedrige gesättigte Heterocyclen, die außerdem als Heteroatome bis zu 2 Sauerstoff-, Schwefel- und/oder Stickstoffatome enthalten können, wie beispielsweise Piperidyl, Morpholinyl oder Piperazinyl oder Pyrrolidinyl. Besonders bevorzugt sind Piperidyl, Morpholinyl und Pyrrolidinyl.

Hydroxyschutzgruppe im Rahmen der oben angegebenen Definition steht im allgemeinen für eine Schutzgruppe aus der Reihe: Trimethylsilyl, Triisopropylsilyl, tert.Butyl-dimethylsilyl, Benzyl, Benzyloxycarbonyl, 2-Nitrobenzyl, 4-Nitrobenzyl, tert. Butyloxycarbonyl, Allyloxycarbonyl, 4-Methoxybenzyl, 4-Methoxybenzyloxycarbonyl, Tetrahydropyranyl, Formyl, Acetyl, Trichloracetyl, 2,2,2-Trichlorethoxycarbonyl, Methoxyethoxymethyl, [2-(Trimethylsilyl)ethoxy]methyl, Benzoyl, 4-Methylbenzoyl, 4-Nitrobenzoyl, 4-Fluorbenzoyl, 4-Chlorbenzoyl oder 4-Methoxybenzoyl. Bevorzugt sind Acetyl, tert. Butyldimethylsilyl und Tetrahydropyranyl.

Aminoschutzgruppen im Rahmen der Erfindung sind die üblichen in der Peptid-Chemie verwendeten Aminoschutzgruppen.

Hierzu gehören bevorzugt: Benzyloxycarbonyl, 2,4-Dimethoxybenzyloxycarbonyl, 4-Methoxybenzyloxycarbonyl, Methoxycarbonyl, Ethoxycarbonyl, tert.Butoxycarbonyl, Allyloxycarbonyl, Phthaloyl, 2,2,2-Trichlorethoxycarbonyl, Fluorenyl-9-methoxycarbonyl, Formyl, Acetyl, 2-Chloracetyl, 2,2,2-Trifluoracetyl, 2,2,2-Trichloracetyl, Benzoyl, 4-Chlorbenzoyl, 4-Brombenzoyl, 4-Nitrobenzoyl, Phthalimido, Isovaleroyl oder Benzyloxymethylen, 4-Nitrobenzyl, 2,4-Dinitrobenzyl, 4-Nitrophenyl, 4-Methoxyphenyl oder Triphenylmethyl.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren als auch deren jeweiligen Mischungen. Die Racemformen lassen sich ebenso wie die Diastereomeren in bekannter Weise in die stereoisomer einheitlichen Bestandteile trennen.

Bevorzugt sind Verbindungen der allgemeinen Formel (I),
in welcher
- A: für jeweils über ein Kohlenstoffatom gebundenes Chinolyl, Benzothiophenyl, Benzthiazolyl, Benzoxazolyl, Pyridyl, Pyridazyl oder Thienyl steht, die gegebenenfalls bis zu 3-fach gleich oder verschieden durch Fluor, Chlor, Brom, Phenyl oder durch geradkettiges oder verzweigtes Alkyl oder Alkylthio mit jeweils bis zu 4 Kohlenstoffatomen oder durch geradkettiges oder verzweigtes Alkenyl mit bis zu 4 Kohlenstoffatomen substituiert sind, das seinerseits durch Phenyl substituiert sein kann, und/oder durch Pyridyl substituiert sind, das seinerseits durch geradkettiges oder verzweigtes Alkyl oder Alkoxy mit jeweils bis zu 5 Kohlenstoffatomen substituiert sein kann, oder
für einen Rest der Formel 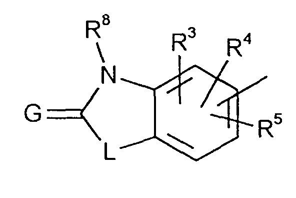 steht,
worin
G ein Sauerstoff- oder Schwefelatom bedeutet,
L ein Sauerstoff- oder Schwefelatom bedeuet,
R⁸ geradkettiges oder verzweigtes Alkyl oder Alkylthio mit jeweils bis zu 6 Kohlenstoffatomen bedeutet,
R³, R⁴ und R⁵ gleich oder verschieden sind und Wasserstoff, Fluor, Chlor oder Brom bedeuten,
- R¹: für einen Rest der Formel 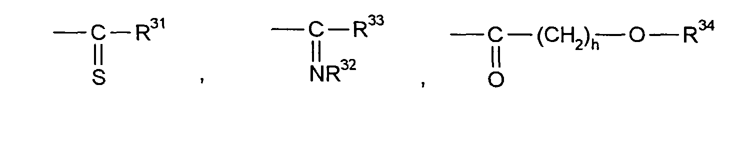 oder 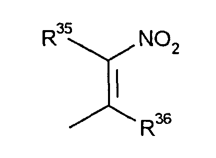 steht,
worin
R³¹ geradkettiges oder verzweigtes Alkyl mit bis zu 5 Kohlenstoffatomen, Cyclopropyl, Cyclobutyl, Cyclopentyl, Cyclohexyl, Phenyl oder eine Gruppe der Formel -NR³⁸R³⁹ bedeutet,
worin
R³⁸ und R³⁹ gleich oder verschieden sind und Wasserstoff, Methyl oder Ethyl bedeuten,
R³² Wasserstoff, Cyano, Cyclopropyl, Cyclobutyl, Cyclopentyl, Cyclohexyl, Phenyl oder geradkettiges oder verzweigtes Alkyl mit bis zu 5 Kohlenstoffatomen bedeutet,
R³³ Wasserstoff, geradkettiges oder verzweigtes Alkyl mit bis zu 5 Kohlenstoffatomen, Phenyl, Cyclopropyl, Cyclobutyl, Cyclopentyl, Cyclohexyl oder eine Gruppe der Formel -NR⁴⁰R⁴¹ bedeutet,
worin
R⁴⁰ und R⁴¹ die oben angegebene Bedeutung von R³⁸ und R³⁹ haben und mit dieser gleich oder verschieden sind,
h eine Zahl 1, 2, 3 oder 4 bedeutet,
R³⁴ Wasserstoff, geradkettiges oder verzweigtes Alkyl mit bis zu 4 Kohlenstoffatomen oder Benzyl bedeutet,
R³⁵ und R³⁶ gleich oder verschieden sind und Wassserstoff oder geradkettiges oder verzweigtes Alkyl mit bis zu 4 Kohlenstoffatomen bedeuten, oder
- R¹: für Cyano oder für Thienyl, Oxazolyl, Thiazolyl, Isoxazolyl oder Pyrazolyl steht, die gegebenenfalls, auch über eine N-Funktion, bis zu 2-fach gleich oder verschieden durch Benzyl, Fluor, Chlor, Brom oder durch geradkettiges oder verzweigtes Alkyl mit bis zu 3 Kohlenstoffatomen substituiert sind,
und deren Salze.

Besonders bevorzugt sind erfindungsgemäße Verbindungen der allgemeinen Formel (I),
in welcher
- A: für jeweils über ein Kohlenstoffatom gebundenes Chinolyl, Benzothiophen, Benzthiazolyl, Benzoxazolyl, Pyridyl, Pyridazyl oder Thienyl steht, die gegebenenfalls bis zu 2-fach gleich oder verschieden durch Fluor, Chlor, Brom, Phenyl, durch geradkettiges oder verzweigtes Alkyl oder Alkylthio mit jeweils bis zu 3 Kohlenstoffatomen oder durch geradkettiges oder verzweigtes Alkenyl mit bis zu 3 Kohlenstoffatomen substituiert sind, das seinerseits durch Phenyl substituiert sein kann, und/oder durch Pyridyl substituiert sind, das seinerseits durch geradkettiges oder verzweigtes Alkyl oder Alkoxy mit jeweils bis zu 4 Kohlenstoffatomen substituiert sein kann, oder
für einen Rest der Formel 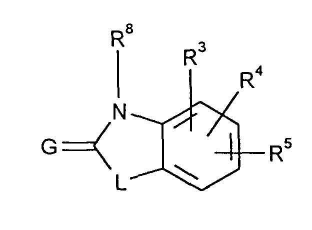 steht,
worin
G ein Sauerstoff- oder Schwefelatom bedeutet,
L ein Sauerstoff- oder Schwefelatom bedeuet,
R⁸ geradkettiges oder verzweigtes Alkyl mit bis zu 4 Kohlenstoffatomen bedeutet,
R³, R⁴ und R⁵ gleich oder verschieden sind und Wasserstoff, Fluor, Chlor oder Brom bedeuten,
- R¹: für einen Rest der Formel 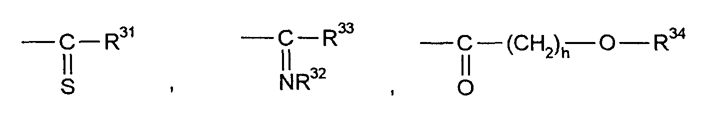 oder 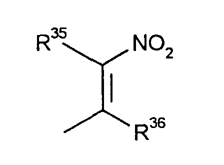 steht,
worin
R³¹ geradkettiges oder verzweigtes Alkyl mit bis zu 4 Kohlenstoffatomen, Cyclopropyl, Cyclobutyl, Cyclopentyl, Cyclohexyl, Phenyl oder eine Gruppe der Formel -NR³⁸R³⁹ bedeutet,
worin
R³⁸ und R³⁹ gleich oder verschieden sind und Wasserstoff oder
Methyl bedeuten,
R³² Wasserstoff, Cyano, Cyclopropyl, Cyclobutyl, Cyclopentyl, Cyclohexyl, Phenyl oder geradkettiges oder verzweigtes Alkyl mit bis zu 4 Kohlenstoffatomen bedeutet,
R³³ Wasserstoff, geradkettiges oder verzweigtes Alkyl mit bis zu 4 Kohlenstoffatomen, Phenyl, Cyclopropyl, Cyclobutyl, Cyclopentyl, Cyclohexyl oder eine Gruppe der Formel -NR⁴⁰R⁴¹ bedeutet,
worin
R⁴⁰ und R⁴¹ die oben angegebene Bedeutung von R³⁸ und R³⁹ haben und mit dieser gleich oder verschieden sind,
h eine Zahl 1, 2, 3 oder 4 bedeutet,
R³⁴ Wasserstoff, geradkettiges oder verzweigtes Alkyl mit bis zu 3 Kohlenstoffatomen oder Benzyl bedeutet,
R³⁵ und R³⁶ gleich oder verschieden sind und Wassserstoff oder geradkettiges oder verzweigtes Alkyl mit bis zu 3 Kohlenstoffatomen bedeuten, oder
- R¹: für Cyano oder für Thienyl, Thiazolyl, Isoxazolyl oder Pyrazolyl steht, die gegebenenfalls, auch über eine N-Funktion, bis zu 2-fach gleich oder verschieden durch Benzyl, Fluor, Chlor, Brom oder durch geradkettiges oder verzweigtes Alkyl mit bis zu 3 Kohlenstoffatomen substituiert sein kann,
und deren Salze.

Weiterhin sind besonders bevorzugt Verbindungen der allgemeinen Formel (I)
in welcher
- A: für jeweils über ein Kohlenstoffatom gebundenes Chinolyl, Pyridyl oder Pyridazyl steht, die gegebenenfalls wie vorstehend angegeben substituiert sind.

Weiterhin sind besonders bevorzugt Verbindungen der allgemeinen Formel (I)
in welcher
- A: für jeweils über ein Kohlenstoffatom gebundenes Benzothiophenyl oder Thienyl steht, die gegebenenfalls wie vorstehend angegeben substituiert sind.

Weiterhin sind besonders bevorzugt Verbindungen der allgemeinen Formel (I)
in welcher
- A: für jeweils über ein Kohlenstoffatom gebundenes Benzothiazolyl oder Benzoxazolyl steht, die gegebenenfalls wie vorstehend angegeben substituiert sind.

Weiterhin sind besonders bevorzugt Verbindungen der allgemeinen Formel (I)
in welcher
- A: für einen Rest der Formel 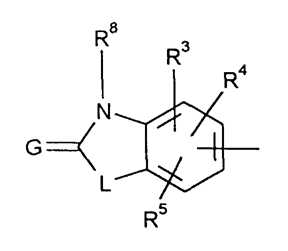 steht, worin R³, R⁴, R⁵ und R⁸ die vorstehend angegebenen Bedeutungen haben können.

Weiterhin sind besonders bevorzugt Verbindungen der allgemeinen Formel (I)
in welcher
- R¹: für einen Rest der Formel 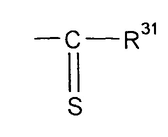 steht,
worin R³¹ wie oben definiert ist.

Weiterhin sind besonders bevorzugt Verbindungen der allgemeinen Formel (I)
in welcher
- R¹: für einen Rest der Formel 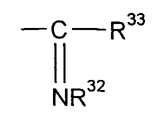 steht,
worin R³² und R³³ wie oben definiert sind.

Weiterhin sind besonders bevorzugt Verbindungen der allgemeinen Formel (I)
in welcher
- R¹: für einen Rest der Formel 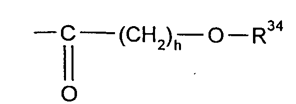 steht,
worin h und R³⁴ wie oben definiert sind.

Weiterhin sind besonders bevorzugt Verbindungen der allgemeinen Formel (I)
in welcher
- R¹: für einen Rest der Formel 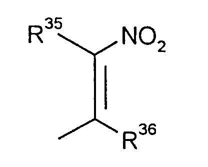 steht,
worin R³⁵ und R³⁶ wie oben definiert sind.

Weiterhin sind besonders bevorzugt Verbindungen der allgemeinen Formel (I)
in welcher
- R¹: für Cyano oder für Thienyl, Thiazolyl, Isoxazolyl oder Pyrazolyl steht, die gegebenenfalls, auch über eine N-Funktion, bis zu 2fach gleich oder verschieden durch Benzyl, Fluor, Chlor, Brom oder durch geradkettiges oder verzweigtes Alkyl mit bis zu 3 Kohlenstoffatomen substituiert sein kann.

Außerdem wurde ein Verfahren zur Herstellung der erfindungsgemäßen Verbindungen der allgemeinen Formel (I) gefunden, dadurch gekennzeichnet, daß man
[A] Verbindungen der allgemeinen Formel (II) 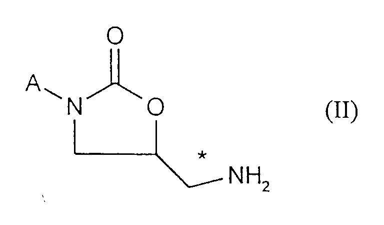 in welcher
   - A: die oben angegebene Bedeutung hat,
   mit Verbindungen der allgemeinen Formel (III)

   R¹-Y (III)

   in welcher
   - R¹: die oben angegebene Bedeutung hat,
   und
   - Y: in Abhängigkeit von R¹ für Wasserstoff, Halogen oder für C₁-C₄ geradkettiges oder verzweigtes Alkoxy, Oxyalkoxycarbonyl oder Pyridyl steht.
   oder
[B] Verbindungen der allgemeinen Formel (IV) 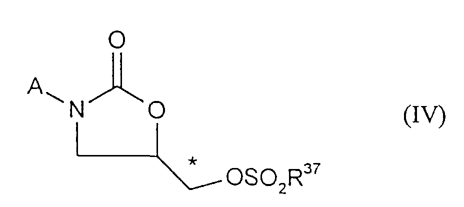 in welcher
   - A: die oben angegebene Bedeutung hat,
   - R³⁷: für C₁-C₄-Alkyl steht,
   mit Verbindungen der allgemeinen Formel (V)

   NH₂-R^{1'} (V)

   in welcher
   - R¹': für einen der oben unter R¹ aufgeführten Heterocyclen steht,
   oder mit Ethyldithioacetat in inerten Lösemitteln, gegebenenfalls in Anwesenheit einer Base umsetzt,
   und im Fall der S-Oxide eine Oxidation nach üblicher Methode durchführt,
   und gegebenenfalls weitere Substituenten oder bereits vorhandene funktionelle Gruppen nach üblichen Methoden, wie beispielsweise Alkylierung, Redoxreaktionen, Substitutionsreaktionen und/oder Verseifungen oder Ein- und Abbau von Schutzgruppen, einführt bzw. derivatisiert.

Die erfindungsgemäßen Verfahren können durch folgende Formelschemata beispielhaft erläutert werden:

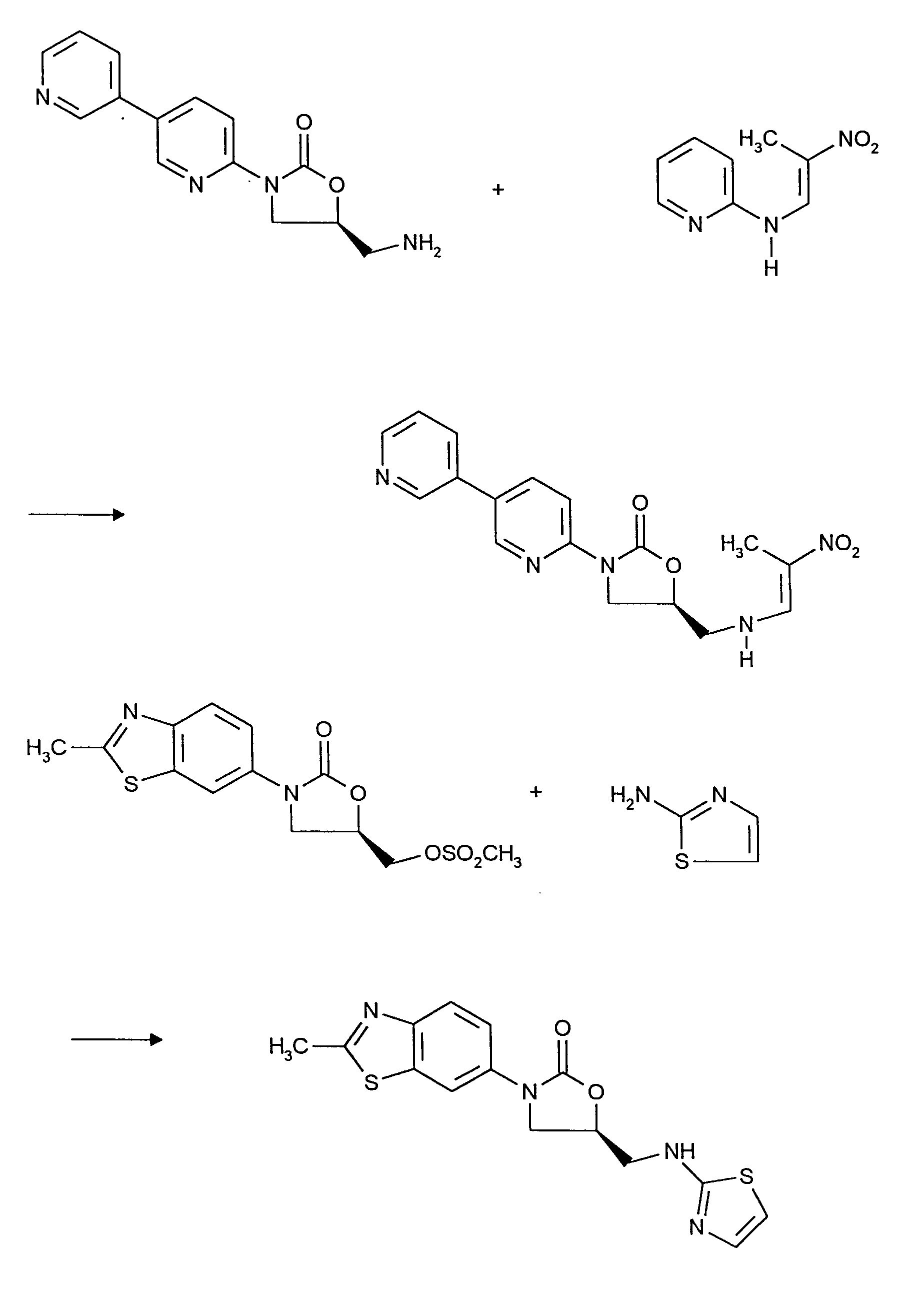

Als Lösemittel eignen sich in Abhängigkeit von den einzelnen Verfahrensschritten die üblichen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, 1,2-Dimethoxyethan, Tetrahydrofuran, Glykoldimethylether oder tert.Butylmethylether, oder Ketone wie Aceton oder Butanon, oder Amide wie Dimethylformamid oder Hexamethyl-phosphorsäuretriamid, oder Kohlenwasserstoffe wie Hexan, Benzol, Dichlorbenzol, Xylol oder Toluol, oder Dimethylsulfoxid, Acetonitril, Essigester, oder Halogenkohlenwasserstoffe wie Methylenchlorid, Chloroform oder Tetrachlorkohlenstoff, oder Pyridin, Picolin oder N-Methylpiperidin. Ebenso können Gemische der genannten Lösemittel verwendet werden.

Als Basen eignen sich in Abhängigkeit von den einzelnen Verfahrensschritten die üblichen anorganischen oder organischen Basen. Hierzu gehören bevorzugt Alkalihydroxide wie beispielsweise Natrium- oder Kaliumhydroxid, oder Alkalicarbonate wie Natrium- oder Kaliumcarbonat, oder Alkalialkoholate wie beispielsweise Natrium- oder Kaliummethanolat, oder Natrium- oder Kaliumethanolat, oder organische Amine wie Ethyldiisopropylamin, Triethylamin, Picolin, Pyridine oder N-Methylpiperidin, oder Amide wie Natriumamid oder Lithiumdiisopropylamid, oder Lithium-N-silylalkylamide, wie beispielsweise Lithium-N-(bis)triphenylsilylamid oder Lithiumalkyle wie n-Butyllithium.

Die Base wird in einer Menge von 1 mol bis 10 mol, bevorzugt von 1 mol bis 3 mol jeweils bezogen auf 1mol der Verbindungen der allgemeinen Formeln (II) und (IV) eingesetzt.

Alle Umsetzungen werden im allgemeinen bei normalem, erhöhtem oder bei erniedrigtem Druck durchgeführt (z.B. 0,5 bis 5 bar). Im allgemeinen arbeitet man bei Normaldruck.

Die Verbindungen der allgemeinen Formeln (III) und (V) sind an sich bekannt oder nach üblichen Methoden herstellbar.

Die Verbindungen der allgemeinen Formel (II) sind teilweise neu und können hergestellt werden, indem man

Verbindungen der allgemeinen Formel (VI) 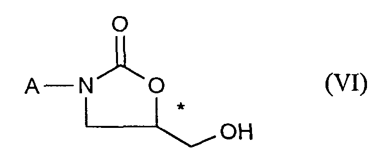 in welcher
- A: die oben angegebene Bedeutung hat,
durch Umsetzung mit (C₁-C₄)-Alkyl- oder Phenylsulfonsäurechloriden in inerten Lösemitteln und in Anwesenheit einer Base in die entsprechenden Verbindungen der allgemeinen Formel (IV) 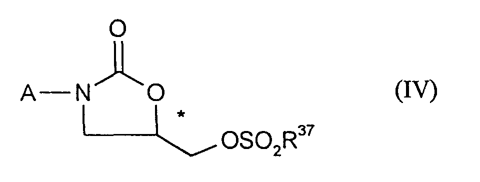 in welcher
- A und R³⁷: die oben angegebene Bedeutung haben
überführt,
anschließend mit Natriumazid in inerten Lösemitteln die Azide der allgemeinen Formel (VII) 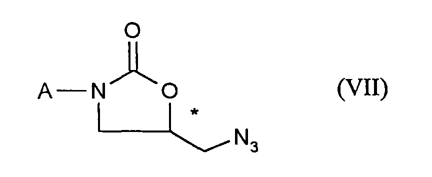 in welcher
- A: die oben angegebene Bedeutung hat,
herstellt,
in einem weiteren Schritt durch Umsetzung mit (C₁-C₄-O)₃-P oder PPh₃, vorzugsweise (CH₃O)₃P in inerten Lösemitteln und mit Säuren in die Amine überführt.

Als Lösemittel eignen sich in Abhängigkeit von den einzelnen Verfahrensschritten die üblichen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, 1,2-Dimethoxyethan, Tetrahydrofuran, Glykoldimethylether oder tert.Butylmethylether, oder Ketone wie Aceton oder Butanon, oder Amide wie Dimethylformamid oder Hexamethyl-phosphorsäuretriamid, oder Kohlenwasserstoffe wie Hexan, Benzol, Dichlorbenzol, Xylol oder Toluol, oder Dimethylsulfoxid, Acetonitril, Essigester, oder Halogenkohlenwasserstoffe wie Methylenchlorid, Chloroform oder Tetrachlorkohlenstoff, oder Pyridin, Picolin oder N-Methylpiperidin. Ebenso können Gemische der genannten Lösemittel verwendet werden.

Als Basen eignen sich in Abhängigkeit von den einzelnen Verfahrensschritten die üblichen anorganischen oder organischen Basen. Hierzu gehören bevorzugt Alkalihydroxide wie beispielsweise Natrium- oder Kaliumhydroxid, oder Alkalicarbonate wie Natrium- oder Kaliumcarbonat, oder Alkalialkoholate wie beispielsweise Natrium- oder Kaliummethanolat, oder Natrium- oder Kaliumethanolat, oder organische Amine wie Ethyldiisopropylamin, Triethylamin, Picolin, Pyridin oder N-Methylpiperidin, oder Amide wie Natriumamid oder Lithiumdiisopropylamid, oder Lithium-N-silylalkylamide, wie beispielsweise Lithium-N-(bis)triphenylsilylamid oder Lithiumalkyle wie n-Butyllithium.

Die Base wird in einer Menge von 1 mol bis 10 mol, bevorzugt von 1 mol bis 3 mol bezogen auf 1mol der Verbindungen der allgemeinen Formel (VI) eingesetzt.

Alle Umsetzungen werden im allgemeinen bei normalem, erhöhtem oder bei erniedrigtem Druck durchgeführt (z.B. 0,5 bis 5 bar). Im allgemeinen arbeitet man bei Normaldruck.

Die Reduktion der Azide erfolgt mit (CH₃O)₃P und Salzsäure.

Die Reduktion erfolgt im allgemeinen in einem Temperaturbereich von -50°C bis zum jeweiligen Siedepunkt des Lösemittels, bevorzugt von -20°C bis +90°C.

Als Lösemittel eignen sich hierbei alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether oder Amide wie Hexamethylphosphorsäuretriamid oder Dimethylformamid, oder Essigsäure. Ebenso ist es möglich, Gemische der genannten Lösemittel zu verwenden.

Die Verbindungen der allgemeinen Formeln (IV) und (VII) sind neu und können wie oben beschrieben hergestellt werden.

Die Verbindungen der allgemeinen Formel (VI) sind teilweise neu und können hergestellt werden, indem man
[D] Verbindungen der allgemeinen Formel (VIII) oder (IX)

   A-N=C=O (VIII) oder A-CO-N₃ (IX)

   in welchen
   - A: die oben angegebene Bedeutungen hat,
   mit Lithiumbromid/(C₄H₉)₃ P(O) und Epoxiden der allgemeinen Formel (X) 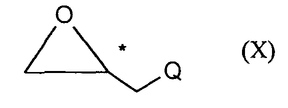 in welcher
   - Q: für C₁-C₆-Acyloxy steht,
   in inerten Lösemitteln, gegebenenfalls in Anwesenheit einer Base
   umsetzt,
   durch eine typische Esterverseifung oder durch eine typische Umesterung die Hydroxyfunktion freisetzt,
   oder
[E] Verbindungen der allgemeinen Formel (XI)

   A-NH-CO₂-X (XI)

   in welcher
   - A: die oben angegebene Bedeutung hat
   und
   - X: für eine typische Schutzgruppe, vorzugsweise Benzyl steht,
   in inerten Lösemitteln und in Anwesenheit einer Base, beispielsweise Lithiumalkylen oder Lithium-N-alkyl- oder Lithium-N-silylalkylamiden, vorzugsweise n-Butyllithium, mit Epoxiden der allgemeinen Formel (X) umsetzt,
   oder
   zunächst Verbindungen der allgemeinen Formel (IX) durch Abspaltung von Stickstoff in Alkoholen in die Verbindungen der allgemeinen Formel (XIa)

   A-NH-CO₂-Y (XIa)

   in welcher
   - A: die oben angegebene Bedeutung hat
   und
   - Y: für geradkettiges oder verzweigtes C₂-C₆-Alkyl, vorzugsweise n-Butyl steht,
   überführt,
   und in einem zweiten Schritt wie unter [D] beschrieben in inerten Lösemitteln und in Anwesenheit einer Base, vorzugsweise Lithium-N-alkyl- oder N-Silylalkylamiden oder n-Butyllithium und Epoxiden der allgemeinen Formel (X) umsetzt,
   oder
[F] Verbindungen der allgemeinen Formel (XII) 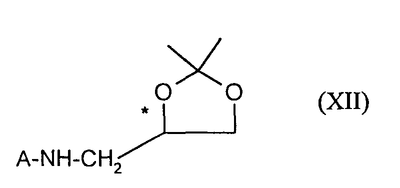 in welcher
   - A: die oben angegebene Bedeutung hat,
   entweder direkt mit Säuren und Kohlensäurediethylester
   umsetzt,
   oder zunächst durch Umsetzung der Verbindungen der allgemeinen Formel (XII) mit Säuren die Verbindungen der allgemeinen Formel (XIII) 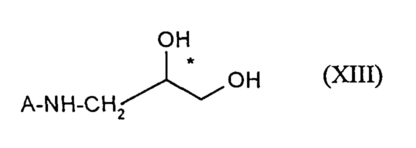 in welcher
   - A: die oben angegebene Bedeutung hat,
   herstellt,
und anschließend in Anwesenheit eines Hilfsmittels in inerten Lösemitteln cyclisiert.

Als Lösemittel eignen sich in Abhängigkeit von den einzelnen Verfahrensschritten die üblichen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, 1,2-Dimethoxyethan, Tetrahydrofuran, Glykoldimethylether oder tert.Butylmethylether, oder Ketone wie Aceton oder Butanon, oder Amide wie Dimethylformamid oder Hexamethyl -phosphorsäuretriamid, oder Kohlenwasserstoffe wie Hexan, Benzol, Dichlorbenzol, Xylol oder Toluol, oder Dimethylsulfoxid, Acetonitril, Essigester, oder Halogenkohlenwasserstoffe wie Methylenchlorid, Chloroform oder Tetrachlorkohlenstoff, oder Pyridin, Picolin oder N-Methylpiperidin. Ebenso können Gemische der genannten Lösemittel verwendet werden.

Als Basen eignen sich in Abhängigkeit von den einzelnen Verfahrensschritten die üblichen anorganischen oder organischen Basen. Hierzu gehören bevorzugt Alkalihydroxide wie beispielsweise Natrium- oder Kaliumhydroxid, oder Alkalicarbonate wie Natrium- oder Kaliumcarbonat, oder Alkalialkoholate wie beispielsweise Natrium- oder Kaliummethanolat, oder Natrium- oder Kaliumethanolat, oder organische Amine wie Ethyldiisopropylamin, Triethylamin, Picolin, Pyridin oder N-Methylpiperidin, oder Amide wie Natriumamid oder Lithiumdiisopropylamid, oder Lithium-N-silylalkylamide, wie beispielsweise Lithium-N-(bis)triphenylsilylamid oder Lithiumalkyle wie n-Butyllithium.

Die Base wird in einer Menge von 1 mol bis 10 mol, bevorzugt von 1 mol bis 3 mol bezogen auf 1mol der Verbindungen der allgemeinen Formeln (X) und (XI) eingesetzt.

Alle Umsetzungen werden im allgemeinen bei normalem, erhöhtem oder bei erniedrigtem Druck durchgeführt (z.B. 0,5 bis 5 bar). Im allgemeinen arbeitet man bei Normaldruck.

Das Verfahren [D] erfolgt bevorzugt in Xylol oder Dichlorbenzol, gegebenenfalls in Gegenwart von Triethylamin, unter Rückfluß.

Die basenkatalysierte Umesterung wird mit einem der oben aufgeführten Alkohole, vorzugsweise Methanol, in einem Temperaturbereich von -10°C bis +40°C, vorzugsweise bei Raumtemperatur durchgeführt.

Als Basen eignen sich im allgemeinen Natriumhydrogencarbonat, Natriummethanolat, Hydrazinhydrat, Kaliumcarbonat oder Caesiumcarbonat. Bevorzugt ist Caesiumcarbonat.

Das Verfahren [E] erfolgt in einem der oben aufgeführten Ether mit Lithiumalkylverbindungen oder Lithium-N-silylamiden, wie beispielsweise n-Butyllithium, Lithiumdiisopropylamid oder Lithium-bistrimethylsilylamid, vorzugsweise in Tetrahydrofuran und Lithium-bis -trimethylsilylamid oder n-Butyllithium, in einem Temperaturbereich von -100°C bis +20°C, vorzugsweise von -75°C bis -40°C.

Für das Verfahren [F] eignen sich für den 1. Schritt vorzugsweise die oben aufgeführten Alkohole, im Falle der anschließenden Cyclisierung Tetrahydrofuran.

Als Basen für die Cyclisierung eignen sich vorzugsweise die oben aufgeführten Lithium-N-silylalkylverbindungen oder n-Butyllithium. Besonders bevorzugt ist n-Butyllithium.

Der erste Reaktionsschritt wird bei der Siedetemperatur des entsprechenden Alkohols, die Cyclisierung in einem Temperaturbereich von -70°C bis Raumtemperatur durchgeführt.

Die Cyclisierung [F] wird in Anwesenheit eines Hilfsmittels und/oder Anwesenheit einer Säure durchgeführt.

Als Säuren eignen sich im allgemeinen anorganische Säuren wie beispielsweise Salzsäure oder Schwefelsäure, oder organische Carbonsäuren mit 1-6 C-Atomen, gegebenenfalls substituiert durch Fluor, Chlor und/oder Brom, wie beispielsweise Essigsäure, Trifluoressigsäure, Trichloressigsäure oder Propionsäure, oder Sulfonsäuren mit C₁-C₄-Alkylresten oder Arylresten, wie beispielsweise Methansulfonsäure, Ethansulfonsäure, Benzolsulfonsäure oder Toluolsulfonsäure. Besonders bevorzugt ist Salzsäure.

Die Säure wird in einer Menge von 1 mol bis 10 mol, bevorzugt von 1 mol bis 2 mol, bezogen auf 1 mol der Verbindungen der allgemeinen Formel (XII) eingesetzt. Als Hilfsmittel eignen sich die üblichen Reagenzien wie Phosgen, Carbonyldiimidazol oder Kohlensäurediethylester oder Chlorameisensäuretrichlormethylester. Bevorzugt sind Carbonyldiimidazol, Kohlensäurediethylester und Chlorameisensäuretrichlormethylester.

Als Lösemittel eignen sich die oben aufgeführten Halogenkohlenwasserstoffe. Bevorzugt ist Methylenchlorid.

Die Verbindungen der allgemeinen Formel (IX) sind bekannt oder können nach üblichen Methoden hergestellt werden.

Die Verbindungen der allgemeinen Formel (XIII) sind größtenteils neu und können beispielsweise wie oben beschrieben hergestellt werden.

Die Verbindungen der allgemeinen Formel (VIII) sind teilweise bekannt oder neu und können dann beispielsweise hergestellt werden, indem man die entsprechenden Amine mit Chlorameisensäuretrichlorethylester in einem der oben aufgeführten Lösemittel, vorzugsweise Xylol bei Rückflußtemperatur umsetzt.

Die Verbindungen der allgemeinen Formel (IX) sind teilweise bekannt oder neu und können dann beispielsweise hergestellt werden, indem man ausgehend von den entsprechenen Carbonsäuren entweder mit Chlorameisensäureisobutylester / Aceton, Natriumazid/Wasser oder mit Diphenylphosphorylazid / Tetrahydrofuran oder mit Xylol oder Methylenchlorid in Gegenwart einer der oben angegebenen Basen, vorzugsweise Triethylamin, bei -10°C bis Raumtemperatur umsetzt.

Die Verbindungen der allgemeinen Formeln (XI) und (XIa) sind teilweise bekannt oder neu und können entweder durch Abspaltung von Stickstoff aus den entsprechenden Carbonsäureaziden und Umsetzung mit den entsprechenden Alkoholen oder durch Umsetzung der entsprechenden Amine mit Chlorameisensäureestern, vorzugsweise Chlorameisensäurebenzylester in einem der oben aufgeführten Lösemittel, vorzugsweise Tetrahydrofuran oder Dioxan, in einem Temperaturbereich von -10°C bis 200°C, vorzugsweise von 0°C bis 150°C, hergestellt werden.

Die minimalen Hemmkonzentrationen (MHK) wurden per Reihenverdünnungsverfahren auf Iso-Sensitest Agar (Oxoid) bestimmt. Für jede Prüfungssubstanz wurde eine Reihe von Agarplatten hergestellt, die bei jeweils doppelter Verdünnung abfallende Konzentrationen des Wirkstoffes enthielten. Die Agarplatten wurden mit einem Multipoint-Inokulator (Denley) beimpft. Zum Beimpfen wurden Übernachtkulturen der Erreger verwandt, die zuvor so verdünnt wurden, daß jeder Impfpunkt ca. 10⁴ koloniebildende Partikel enthielt. Die beimpften Agarplatten wurden bei 37°C bebrütet, und das Keimwachstum wurde nach ca. 20 Stunden abgelesen. Der MHK-Wert (µg/ml) gibt die niedrigste Wirkstoffkonzentration an, bei der mit bloßem Auge kein Wachstum zu erkennen war.

| **MHK-Werte (µg/ml):** | | | | | | | |
|---|---|---|---|---|---|---|---|
| **Bsp.- Nr.** | **Staph. 133** | **Staph. 48N** | **Staph. 25701** | **Staph. 9TV** | **E. coli Neumann** | **Klebs. 57 USA** | **Psdm. Bonn** |
| 11 | 0.5 | 0.5 | 0.25 | 0.25 | >64 | >64 | >64 |
| 12 | 1 | 1 | 1 | 0.5 | >64 | >64 | >64 |
| 13 | 0.25 | 0.25 | 0.25 | ≤ 0.125 | >64 | >64 | >64 |
| 14 | 1 | 1 | 1 | 0.5 | >64 | >64 | >64 |
| 15 | 0.25 | 0.5 | 0.25 | ≤ 0.125 | >64 | >64 | >64 |
| 20 | 0.25 | 0.25 | 0.25 | ≤ 0.125 | 64 | >64 | >64 |
| 23 | 0.25 | 0.5 | 0.5 | 0.25 | >64 | >64 | >64 |
| 26 | < 0.125 | 0.25 | 0.25 | ≤ 0.125 | >64 | >64 | >64 |
| 42 | < 0.125 | >0.125 | >0.125 | < 0.125 | 64 | 64 | >64 |
| 43 | 0.5 | 0.5 | 0.5 | 0.5 | >64 | >64 | >64 |

Für schnellwachsende Mykobakterien wurde die MHK-Bestimmung in Anlehnung an die von Swenson beschriebene Methode der Bouillon Mikrodilution durchgeführt [vgl. J.M. Swenson, C. Thornberry, U.A. Silcox, Rapidly growing mycobacteria. Testing of susceptibility to 34 antimicrobial agents by broth microdilution. Antimicrobial Agents and Chemotherapy Vol. 22, 186-192 (1982)]. Abweichend davon war das mit 0,1 Vol.% Tween 80 versetzte Hirn-Herzextrakt Medium.

Die verwendeten Mykobakterienstämme wurden von der DSM (Dt. Sammlung von Mikroorganismen, Braunschweig) bezogen. Sie wurden in einer feuchten Kammer bei 37°C bebrütet.

Die MHK-Werte wurden nach 2-4 Tagen abgelesen, wenn die präparatfreien Kontrollen durch Wachstum trüb waren. Der MHK-Wert definiert sich als die niedrigste Präparatkonzentration, die makroskopisch sichtbares Wachstum völlig inhibiert.

| **MHK-Werte: Mycobacterium smegmatis** | | |
|---|---|---|
| **Stamm:** | **DSM 43061** | **DSM 43078** |
| Inoculum [/ml] | 2,20E+04 | 4,20E+04 |

| Bsp.-Nr. | | |
|---|---|---|
| 12 | 8 | 4 |
| 13 | 2 | 1 |
| 14 | 8 | 4 |
| 15 | 1 | 0.5 |
| 20 | 0.25 | 0.25 |
| Isoniazid | 4 | 2 |
| Strepto-mycin | 4 | 4 |

### MHK-Bestimmung mit Mycoplasma pneumoniae

Mycoplasma pneumoniae Stamm PI 1428 wurde unter aeroben Bedingungen in PPLO-Medium, dem 1% Glukose, 2,5% Hefeextrakt, 20% Pferdeserum (donor horse serum) und 0,002% Phenolrot zugegeben wurden gezüchtet. MHK-Bestimmungen wurden in Anlehnung an die von ter Laak u. Mitarbeitern beschriebene Methode der Reihenmikrodilution in Flüssigmedium durchgeführt (E. A. ter Laak, A. Pijpers, J.H. Noordergraaf, E. Schoevers, J.H.M. Verheijden: Comparison of Methods for in vitro Testing of Susceptibility of Porcine Mycoplasma Species to Antimicrobial Agents; Antimicrobial Agents and Chemotherapy, Vol. 35, 228-233 (1991)). Zum Zeitpunkt des beginnenden Farbumschlags des Mediums der präparatfreien Kontrolle von rot nach gelb wurden 10 Vol% Alamar Blau zugegeben. Die Inkubation bei 37°C wurde für ca. 10 Stunden fortgesetzt und die MHK als der Wert definiert, bei dem das Medium mit der kleinsten Präparatkonzentration unverändert blau blieb.

| **Bsp.-Nr.** | **MHK (µg/ml**) |
|---|---|
| 12 | 2 |
| 13 | 2 |
| 14 | 8 |
| 23 | 4 |

Die erfindungsgemäßen Verbindungen der allgemeinen Formel (I) weisen bei geringer Toxizität ein breites antibakterielles Spektrum, speziell gegen gram-positive Bakterien sowie Mycobacterien, Haemophilus influenzae, anaerobe Keime für schnellwachsende Mykobakterien auf. Diese Eigenschaften ermöglichen ihre Verwendung als chemotherapeutische Wirkstoffe in der Human- und Tiermedizin.

Besonders wirksam sind die erfindungsgemäßen Verbindungen gegen Bakterien und bakterienähnliche Mikroorganismen wie Mycoplasmen. Sie sind daher besonders gut zur Prophylaxe und Chemotherapie von lokalen und systemischen Infektionen in der Human- und Tiermedizin geeignet, die durch solche Erreger hervorgerufen werden.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Der oder die Wirkstoffe können gegebenenfalls in einem oder mehreren der oben angegebenen Trägerstoffe auch in mikroverkapselter Form vorliegen.

Die therapeutisch wirksamen Verbindungen sollen in den oben aufgeführten pharmazeutischen Zubereitungen in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30mg/kg Körpergewicht.

Die erfindungsgemäßen Verbindungen können zum Zweck der Erweiterung des Wirkungsspektrums und um eine Wirkungssteigerung zu erreichen auch mit anderen Antibiotika kombiniert werden.

### Anhang zum experimentellen Teil

### Liste der verwendeten Laufmittelgemische zur Chromatographie:

- I: Dichlormethan : Methanol
- II: Toluol : Ethylacetat
- III: Acetonitril : Wasser
- IV: Ethylacetat
- V: Petrolether : Ethylacetat
- VI: CH₂Cl₂:MeOH:NH_{3(aq)}
- VII: CH₂Cl₂ : MeOH

### Abkürzungen:

- Z: Benzyloxycarbonyl
- Boc: tert.Butoxycarbonyl
- DMF: Dimethylformamid
- Ph: Phenyl
- Me: Methyl
- THF: Tetrahydrofuran
- CDI: Carbonyldiimidazol
- DCE: Dichlorethan

Sofern nichts anderes angegeben ist, können die nachfolgend aufgeführten Beispiele nach oder in Analogie zu den Angaben in EP 694 543, EP 693 491, EP 694 544, EP 697 412 und EP 738 726 hergestellt werden.

### Ausgangsverbindungen

### Beispiel I

### 5-Brom-2-isocyanato-pyridin Hydrochlorid

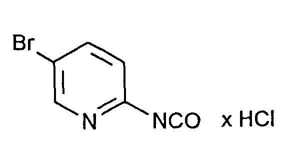

Zu einer gerührten Lösung von 100 g (0,58 mol) 2-Amino-5-brompyridin in 400 ml 1,2-Dichlorethan tropft man in der Siedehitze 78,0 ml (0,64 mol) Chlorameisensäuretrichlorethylester. Nach der Zugabe wird 2h im Rückfluß gekocht, dann darf sich das Gemisch auf Raumtemperatur abkühlen. Der entstandene Niederschlag wird durch Filtration abgetrennt, mit 100 ml 1,2-Dichlorethan gut gewaschen und im Hochvakuum über Natriumhydroxid getrocknet. Man erhält 98,3 g (72%) der Titelverbindung als gelben Feststoff.
Schmp.: 248-254°C (Zers.)
R_{f} = 0,23 (Ethylacetat)
MS (EI) m/z = 198 (M)⁺

### Beispiel II

### (5R)-3-(5-Brom-pyridin-2-yl)-5-butyryloxy-methyl-oxazolidin-2-on

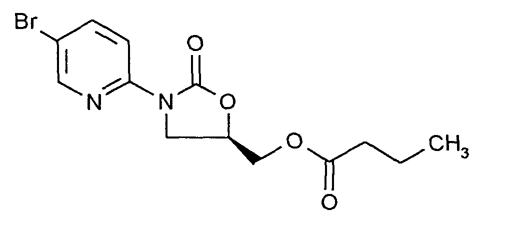

Eine Suspension von 2,17 g (25 mmol) Lithiumbromid und 5,46 g (25 mmol) Tributylphophinoxid in 73 ml Xylol wird 1 h am Wasserabscheider gekocht. Dazu wird in der Siedehitze ein Gemisch von 58,5 ml (0,42 mol) Triethylamin und 66,6 g (0,42mol) (R)-Glycidylbutyrat getropft. Gleichzeitig werden innerhalb von 20 min 98,2 g (0,42 mol) der Verbindung aus Beispiel I portionsweise zugegeben.

Nach beendeter Zugabe wird noch 1 h unter Rückfluß nachgerührt. Man läßt auf Raumtemperatur abkühlen und dampft das Lösemittel im Vakuum ab. Nach Chromatographie des Rückstands an 1 kg Kieselgel (Toluol : Ethylacetat 95:5) erhält man 37,9 g (26%) der Titelverbindung als Öl.
R_{f} = 0,43 (Toluol : Ethylacetat 4:1)
MS (FAB) m/z = 343 (M+H)⁺
¹H-NMR.(250 MHz, D₆-DMSO): δ = 0,81 (t, J = 7 Hz, 3H, CH₃CH₂); 1,5 (m, 2H, CH₃CH₂CH₂CO); 2,29 (t, J = 7 Hz, 2H, CH₃CH₂CH₂CO); 3,91 (dd, J = 7 Hz, 10 Hz, 1H, H-4 trans); 4,25 (dd, J = 9 Hz, 10 Hz, 1H, H-4 cis); 4,36 (m, 2H, CH₂O); 4,97 (m, 1H, H-5); 8,08 (d, J = 1 Hz, 2H, Pyridyl H-3,4); 8,50 (d, J = 1 Hz, pyridyl H-6).

### Beispiel III

### (5R)-3-(5-Brom-pyridin-2-yl)-5-hydroxymethyl-oxazolidin-2-on

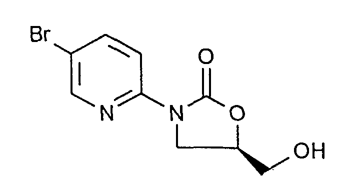

Eine Lösung von 19,6 g (57,3 mmol) der Verbindung aus Beispiel II in 125 ml wasserfreiem Methanol wird mit 185 mg (0,57 mmol) Caesiumcarbonat versetzt und 5 h bei Raumtemperatur gerührt. Das Lösemittel wird im Vakuum abgedampft und der Rückstand wird mit 30 ml Ether verrührt. Der Niederschlag wird durch Filtration abgetrennt, mit 25 ml Wasser und 5 ml Ether gewaschen und im Hochvakuum getrocknet. Man erhält 10,73 g (69%) der Titelverbindung als helle Kristalle.
Schmp.: 0,09 (Toluol : Ethylacetat 4:1)
MS (DCI, NH₃) m/z = 273 (M+H)⁺
¹H-NMR (200 MHz, CD₃OD) δ = 3,68 (d, J = 5,9 Hz, 1 H, CH₂O); 3,87 (dd, J = 4, 9 Hz, 1H, CH₂O); 4,06 (dd, J = 7, 10 Hz, 1H, H-4 trans); 4,26 (dd, J = 9, 10 Hz, 1H, H-4 cis); 4,75 (m, 1H, H-5); 7,92 (dd, J = 1,5 Hz, 10 Hz, 1H, Pyridyl H-3); 8,12 (d, J = 10 Hz, 1H, Pyridyl H-4); 8,40 (d, J = 1,5 Hz, 1H, Pyridyl H-6).

### Beispiel IV

### 5-(2-Pyridyl)-thiophen-2-carbonsäureazid

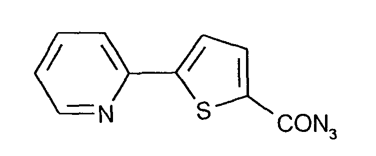

20 g (97,45 mmol) 5-(2-Pyridyl)-thiophen-2-carbonsäure werden in 200 ml Aceton gelöst, mit 15,94 ml (115 mmol) Et₃N versetzt und auf 0°C gekühlt. Zu der so erhaltenen Reaktionslösung tropft man langsam unter Rühren eine Lösung von 14,85 ml (115 mmol) Chlorameisensäureisobutylester in 88 ml Aceton. Nach 1 h bei 0°C tropft man eine Lösung von 9,5 g (146 mmol) Natriumazid in 44 ml Wasser zu, rührt 1 h bei 0°C nach und läßt auf Raumtemperatur kommen. Die Reaktionsmischung wird auf Eiswasser gekippt und abgesaugt und so weiter umgesetzt.
Ausbeute: 21 g wasserfeuchtes Pulver.

### Beispiel V

### 5-(2-Pyridyl)-butyloxycarbonylamino-thiophen

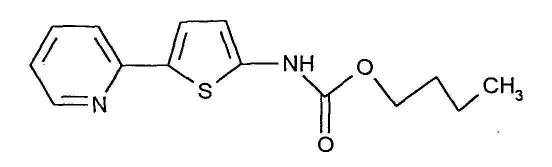

21 g der Verbindung aus Beispiel IV werden portionsweise in 400 ml siedendes n-Butanol eingetragen. Nach beendeter Gasentwicklung wird 15 min unter Rückfluß nachgerührt. Nach Abkühlen auf Raumtemperatur wird eingeengt, der Rückstand mit Ether verrührt, abgesaugt und bei 50°C im Umlufttrockenschrank getrocknet.
Ausbeute: 18,8 g (75% d.Th.)
¹H-NMR (200 MHz, D₆-DMSO): δ = 10,8 (s, 1H); 8,45 (d, J = 5 Hz, 1H); 7,68 - 7,85 (m, 2H); 7,5 (d, J = 5 Hz, 1H); 7,1 - 7,2 (m, 1H); 6,57 (d, J = 5 Hz, 1H); 4,14 (t, J = 7 Hz,, 2H); 1,62 (q, J = 7 Hz, 2H); 1,39 (h, J = 7 Hz, 2H); 0,92 (t, J = 7 Hz, 3H).

### Beispiel VI

### (5R)-3-(5-(2-Pyridyl)-thien-2-yl)-5-hydroxymethyl-oxazolidin-2-on

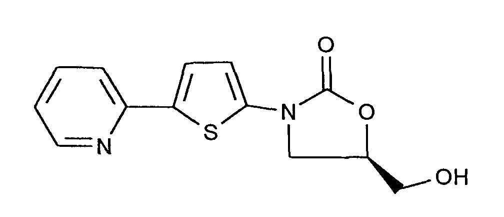

18,8 g (68 mmol) der Verbindung aus Beispiel V werden in 190 ml absolutem THF gelöst, mit 10 mg 1,10-Phenanthrolin-Hydrat verstzt und auf -70°C gekühlt. Nun werden langsam ca. 27 ml 2,5 N n-Butyllithium-Lösung in Hexan bis zum Farbumschlag nach rot zugetropft. Anschließend werden 9,6 ml (68 mmol) (R)-Glycidylbutyrat zugetropft. Man läßt auf Raumtemperatur kommen, versetzt mit gesättigter Ammoniumchloridlösung, trennt die organische Phase ab und extrahiert die wäßrige Phase zweimal mit Methylenchlorid. Die vereinigten organischen Phasen werden getrocknet (Na₂SO₄) und eingeengt. Der Rückstand wird mit Ether verrührt und abgesaugt.
Ausbeute: 15,3 g (81,5% d.Th.)
R_{f} = 0,06 (CH₂Cl₂ : CH₃OH = 100:3)
Smp.: 191°C
¹H-NMR (200 MHz, D₆-DMSO); δ = 8,45 (d, J = 5 Hz, 1H); 7,7 - 7,9 (m, 2H); 7,6 (d, J = 5 Hz, 1H); 7,15 - 7,25 (m, 1H); 6,58 (d, J = 5 Hz, 1H); 5,28 (t, J = 7 Hz, 1H); 4,77 - 4,9 (m, 1H); 4,13 (dd, J = 10 Hz, 9 Hz, 1H); 3,86 (dd, J = 10 Hz, 6 Hz, 1H); 3,55 - 3,78 (m, 2H).

### Beispiel VII

### 6-(Benzyloxycarbonylamino)-3-methyl-2-benzothiazolinon

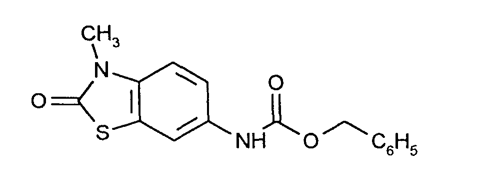

1,76 g (8,12 mmol) 6-Amino-3-methyl-2(3H)-benzothiazolon-hydrochlorid in 17 ml Wasser, 14 ml THF und 17 ml ges. NaHCO₃-Lösung werden bei 0°C tropfenweise mit 1,3 ml (9,10 mmol) Chlor-ameisensäurebenzylester versetzt. Nach 1 h werden 120 ml Wasser hinzugegeben, das THF im Vakuum abgezogen, der Niederschlag abgesaugt, dreimal mit Wasser, zweimal mit Petrolether gewaschen und bei 60°C getrocknet.
Ausbeute: 2,44 g (96%)
Smp.: 183°C
R_{f} (II, 7:3) = 0,39
¹H-NMR ([D₆]DMSO): δ = 7,77 (d, J = 1 Hz, 1H, Benzothiazolinon 7-H); 7,23 - 7,45 (m, 6H, Ph), 7,22 (d, J = 6 Hz, 1H, Benzothiazolinon 4-H); 5,15 (s, 2H); 3 ,38 (s, 3H-CH₃).

### Beispiel VIII

### (5R)-3-[3-Methyl-2-benzothiazolinon-6-yl]-5-(hydroxymethyl)-oxazolidin-2-on

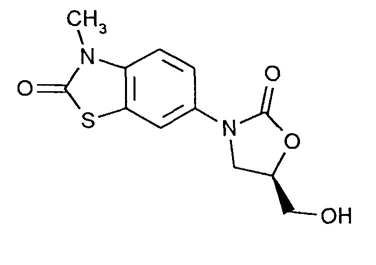

### Methode A

26,76 g (85,12 mmol) der Verbindung aus Beispiel VII werden in 400 ml THF gelöst, mit 10 mg 1,10-Phenanthrolin-Hydrat versetzt und auf -70°C gekühlt. Nun werden langsam ca. 34 ml 2,5 N n-Butyllithium-Lösung in Hexan bis zum Farbumschlag nach rot zugetropft. Anschließend werden 12 ml (85,12 mmol) (R)-Glycidylbutyrat zugetropft. Man läßt auf RT kommen, versetzt mit gesättigter Ammoniumchloridlösung und zieht im Vakuum das THF ab. Der entstandene Niederschlag wird abgesaugt, mit Wasser und Ether gewaschen und im Hochvakuum getrocknet.
Ausbeute: 17,93 g (75%)
Smp.: 166°C
R_{f} (II, 1:1) = 0,09
MS (EI): m/z = 280 (M⁺)
¹H-NMR ([D₆]DMSO): δ = 7,80 (d, J = 1 Hz, 1H, Benzothiazolinon 7-H); 7,60 (dd, J = 6, J = 1 Hz, 1H, Benzothiazolinon 5-H); 7,32 (d, J = 6 Hz, 1H, Benzthiazolinon 4-H); 5,23 (t, J = 6 Hz, 1H, OH); 4,62 - 4,80 (m, 1H, 5-H); 4,10 (t, J = 9 Hz, 1H, 4-H); 3,85 (dd, J = 9, J = 5 Hz, 1H, 4-H); 3,48 - 3,75 (m, 2H, CH₂O); 3,40 (s, 3H, CH₃).

### Methode B

9,3 g (0,03 mol) der Verbindung aus Beispiel VII werden in 150 ml THF gelöst und auf -70°C gekühlt. Anschließend werden 4 ml (0,01 mol) 2,5 M n-Butyllithiumlösung in Hexan zugetropft. Danach werden gleichzeitig langsam nochmals 8 ml (0,02 mol) n-Butyllithium und 4,23 ml (0,03 mol) (R)-Glycidylbutyrat zugetropft. Man läßt auf Raumtemperatur kommen und rührt drei Stunden nach. Die Aufarbeitung erfolgt wie für Methode A beschrieben. Ausbeute: 6 g (72 %).

Analog den Vorschriften der Beispiele I bis VIII werden die in Tabelle I aufgeführten Verbindungen dargestellt:

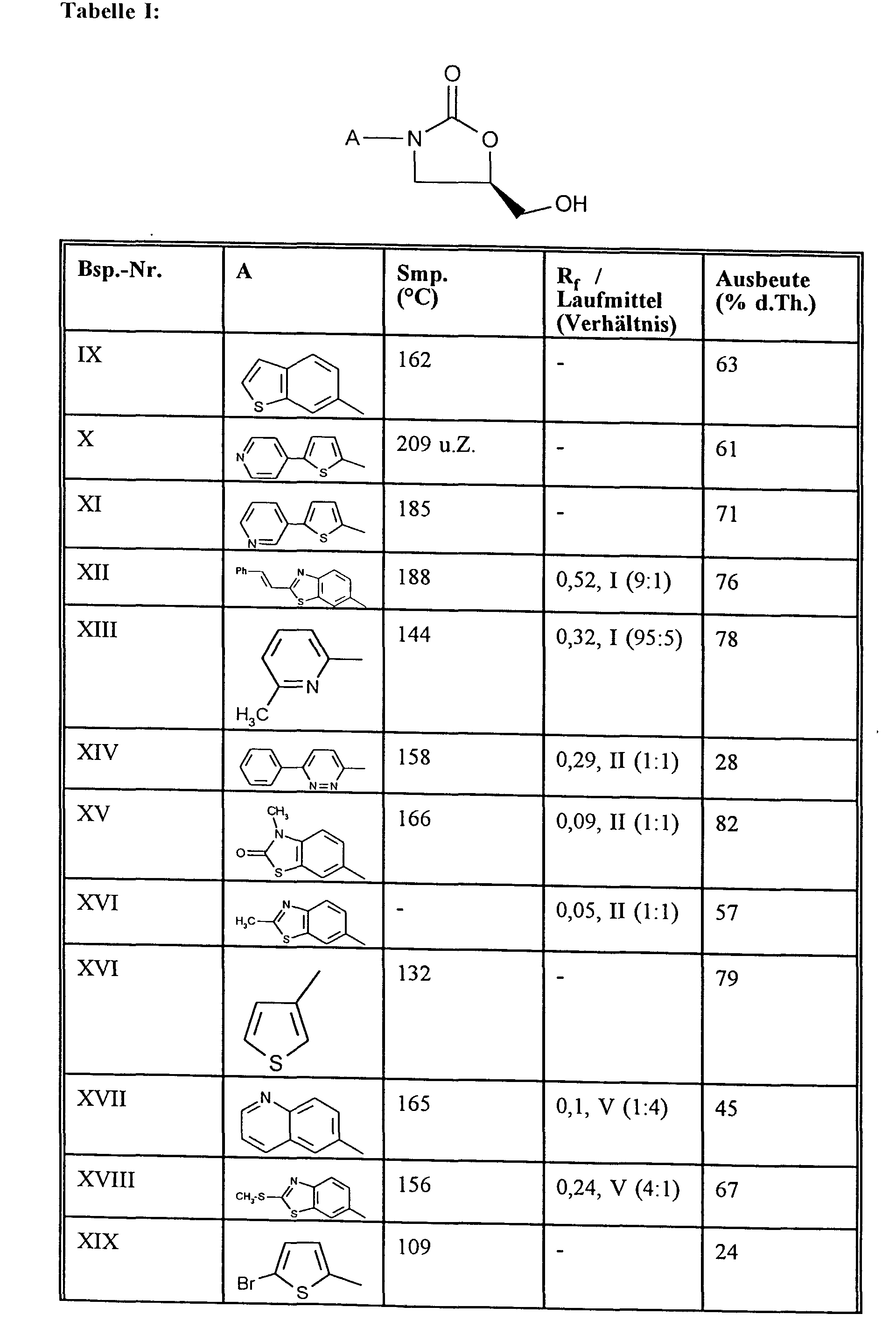

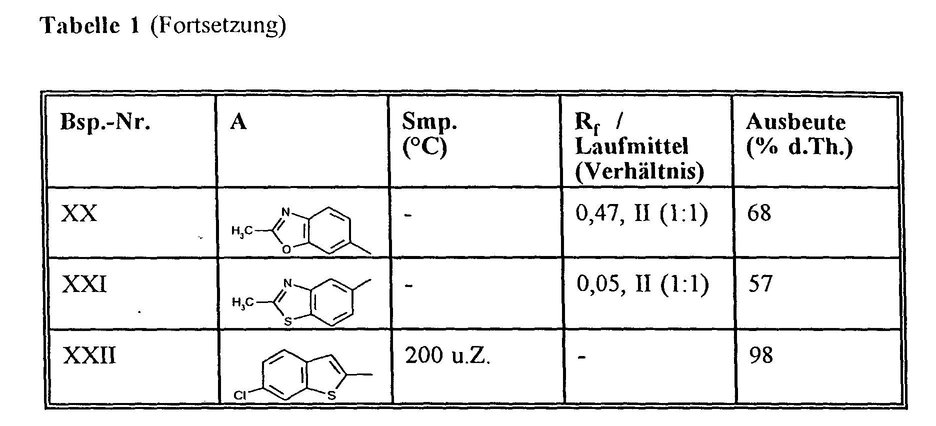

### Beispiel XXIII

### (5R)-3-(5-Brom-pyridin-2-yl)-5-methansulfonyloxy-methyl-oxazolidin-2-on

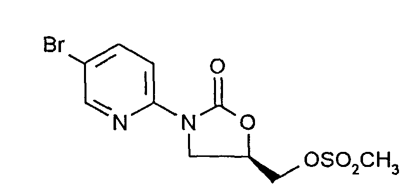

Eine auf 0°C gekühlte, gerührte Lösung von 10,5 g (38,44 mmol) der Verbindung aus Beispiel III und 6,40 ml (46,14 mmol) Triethylamin in 36 ml wasserfreiem Dichlormethan wird langsam mit 3,27 ml (42,28 mmol) Methansulfonsäurechlorid versetzt. Man rührt 10 min. bei 0-5°C nach und rührt das Gemisch in 50 ml Eiswasser ein. Die organische Phase wird abgetrennt, mit 20 ml gesättigter NaHCO₃-Lösung und 20 ml Eiswasser gewaschen und über MgSO₄ getrocknet. Das Lösemittel wird im Vakuum eingedampft und der Rückstand mit 50 ml Ether verrührt, abgesaugt und im Hochvakuum getrocknet. Man erhält 12,8 g (95%) der Titelverbindung als farblose Kristalle.
Schmp.: 138-138,5°C
R_{f} = 0,65 (Dichlormethan : Methanol 95:5)
MS (DCI, NH₃) m/z = 351 (M+H)⁺
¹H-NMR (250 MHz, D₆-DMSO) δ = 3,25 (s, 3H, OSO₂CH₃); 3,91 (dd, J = 7, 10 Hz, 1H, H-4 trans); 4,27 (dd, J = 10, 10 Hz, 1H, H-4 cis); 4,52 (m, 2H, CH ₂O); 5,02 (m, 1H, H-5); 8,09 (s, 2H, Pyridyl H-3,4); 8,52 (s, 1H, Pyridyl H-6).

### Beispiel XXIV

### (5R)-3-(5-Brom-pyridin-2-yl)-5-azidomethyl-oxazolidin-2-on

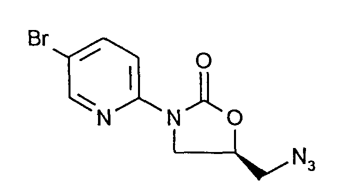

Eine gerührte Lösung von 12,5 g (35,6 mmol) der Verbindung aus Beispiel XXIII in 48 ml wasserfreiem DMF wird mit 3,01 g (46,28 mmol) Natriumazid versetzt und 3 h bei 70°C gerührt. Man läßt auf Raumtemperatur abkühlen und rührt in 100 ml Eiswasser ein. Der entstandene Niederschlag wird durch Filtration abgetrennt, mit 50 ml Wasser und 20 ml Petrolether gewaschen und an der Luft getrocknet. Man erhält 10,1 g (95%) der Titelverbindung als helle Kristalle.
Schmp.: 64-67°C
R_{f} = 0,63 (Toluol : Ethylacetat 2:3)
MS (DCI, NH₃) m/z = 298 (M+H)⁺
¹H-NMR (250 MHz, D₆-DMSO) δ = 3,73 (m, 2H, CH₂N₃); 3,87 (dd, J = 6, 8 Hz, 1H, H-4 trans); 4,22 (dd, J = 8, 8 Hz, 1H, H-4 cis); 4,92 (m, 1H, H-5); 8,08 (s, 2H, Pyridyl H-3,4); 8,51 (s, 1H, Pyridyl H-6).

### Beispiel XXV

### (5S)-3-(5-Brom-pyridin-2-yl)-5-aminomethyl-oxazolidin-2-on Hydrochlorid

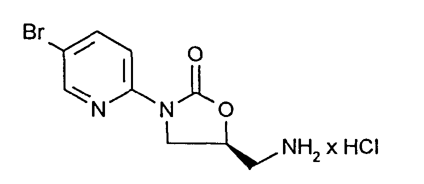

Eine gerührte Lösung von 10,1 g (33,9 mmol) der Verbindung aus Beispiel XXIV in 16,5 ml 1,2-Dimethoxyethan wird auf 50°C erwärmt. Man tropft langsam 4,68 ml (4,70 mmol) Trimethylphosphit zu (Gasentwicklung) und rührt nach beendeter Zugabe nach 2 h bei 90°C nach. Nun tropft man 6,6 ml 6 N HCl zu und rührt nochmals 2 h bei 90°C nach. Man läßt auf Raumtemperatur abkühlen, trennt den Niederschlag durch Filtration ab, wäscht mit 2 x 10 ml 1,2-Dimethoxyethan und trocknet im Hochvakuum über NaOH. Man erhält 8,9 g (85%) der Titelverbindung als farblose Kristalle.
Schmp.: 260-262°C
R_{f} = 0,53 (Acetonitril : Wasser 4:1)
MS (EI) m/z = 271 (M⁺)
¹H-NMR (250 MHz, D₆-DMSO) δ = 3,28 (m, 2H, CH₂NH₂); 3,93 (dd, J 7, 9 Hz, 1H, H-4 trans); 4,28 (dd, J = 9, 9 Hz, 1H, H-4 cis); 5,00 (m, 1H, H-5); 8,05 (s, 2H, Pyridyl H-3,4); 8,5 (m, 3H, NH₂, Pyridyl H-6).

### Beispiel XXVI

### (5S)-3-(5-Brom-pyridin-2-yl)-5-((tert.butyloxy)carbonyl)aminomethyl-oxazolidin-2-on

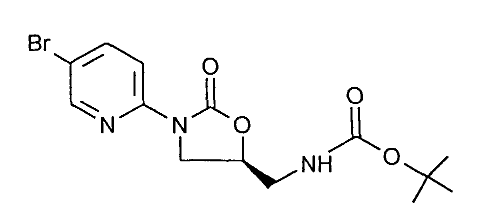

Man suspendiert 4,7 g (15 mmol) der Verbindung aus Beispiel XXV in 100 ml CH₂Cl₂. Anschließend fügt man 2,2 ml (16 mmol) Triethylamin zu, wobei eine Lösung entsteht. Man kühlt auf 0°C ab. Nun addiert man 3,5 g (16 mmol) Boc-Anhydrid so zu, daß die Temperatur +5°C nicht übersteigt und läßt bei Raumtemperatur über Nacht nachrühren. Man wäscht die organische Phase mit ges. NaCl-Lösung, trocknet über MgSO₄ und engt ein. Man erhält 5,4 g (97% d.Th.) des Produktes als weißen Feststoff.
Fp.: 184°C
R_{f}-Wert (Petrolether : Essigester = 10:4) = 0,30

### Beispiel XXVII

### ((5S)-3-(5-[3-Pyridyl]-pyridin-2-yl)-5-((tert.butyloxy)carbonyl)aminomethyloxazolidin-2-on

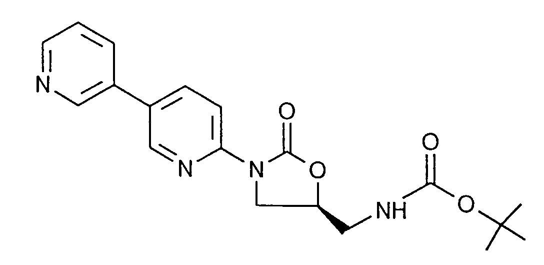

Unter Argon legt man 5,3 g (14,24 mmol) der Verbindung aus Beispiel XXVI und 2,81 g Diethyl-(3-pyridyl)-boran in 100 ml abs. THF vor. Man addiert eine Lösung von 0,5 g (0,43 mmol) [(PPh₃)₄Pd] in 90 ml THF und 4,9 ml (9,83 mmol) 2 M Natriumcarbonatlösung. Man läßt den Ansatz 5 Tage bei Rückfluß rühren. Nach dem Abkühlen auf RT gibt man 10 g Kieselgur zu und engt ein. Der Rückstand wird auf eine mit Kieselgel gefüllte Säule aufgetragen und mit Essigester eluiert.
Man erhält 4 g (76% d.Th.) der Titelverbindung
Fp.: 163°C
R_{f}-Wert = 0,36 (CH₂Cl₂ : MeOH = 100:5)

### Beispiel XXVIII

### (5S)-3-(5-[3-Pyridyl]-pyridin-2-yl)-5-aminomethyl-oxazolidin-2-on Trihydrochlorid

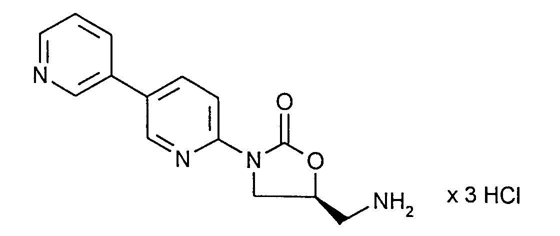

3,8 g (10,3 mmol) der Verbindung aus Beispiel XXVII werden in 25 ml Dioxan suspendiert. Man addiert 32,1 ml einer 4 M HCl-Lösung in Dioxan und läßt über Nacht bei Raumtemperatur rühren. Man engt ein und rührt den Rückstand mit Ether aus. Anschließend wird der Feststoff durch eine Fritte abgesaugt und mit Ether nachgewaschen. Man trocknet am Hochvakuum und erhält 3,7 g (95% d.Th.) der Titelverbindung.
Fp.: >250°C
MS (EI): 271 (M⁺), 172
¹H-NMR (200 MHz, DMSO-d₆); δ = 9,35 (sb, 1H); 8,93 (m, 3H); 8,6 (breit, 3H); 8,42 (dd, J = 9, J = 3, 1H); 8,24 (d, J = 9, 1H); 8,11 (dd, J = 7,5, J = 6,5, 1H); 6,7 - 5,3 (breit, 2H); 5,06 (m, 1H); 4,38 (tr, J = 10, 1H); 4,03 (dd, J = 10, J = 7,5, 1H); 3,29 (m, 2H).

Analog den Vorschriften der Beispiele XXIII bis XXVIII wurden die in Tabelle II aufgeführten Verbindungen dargestellt:

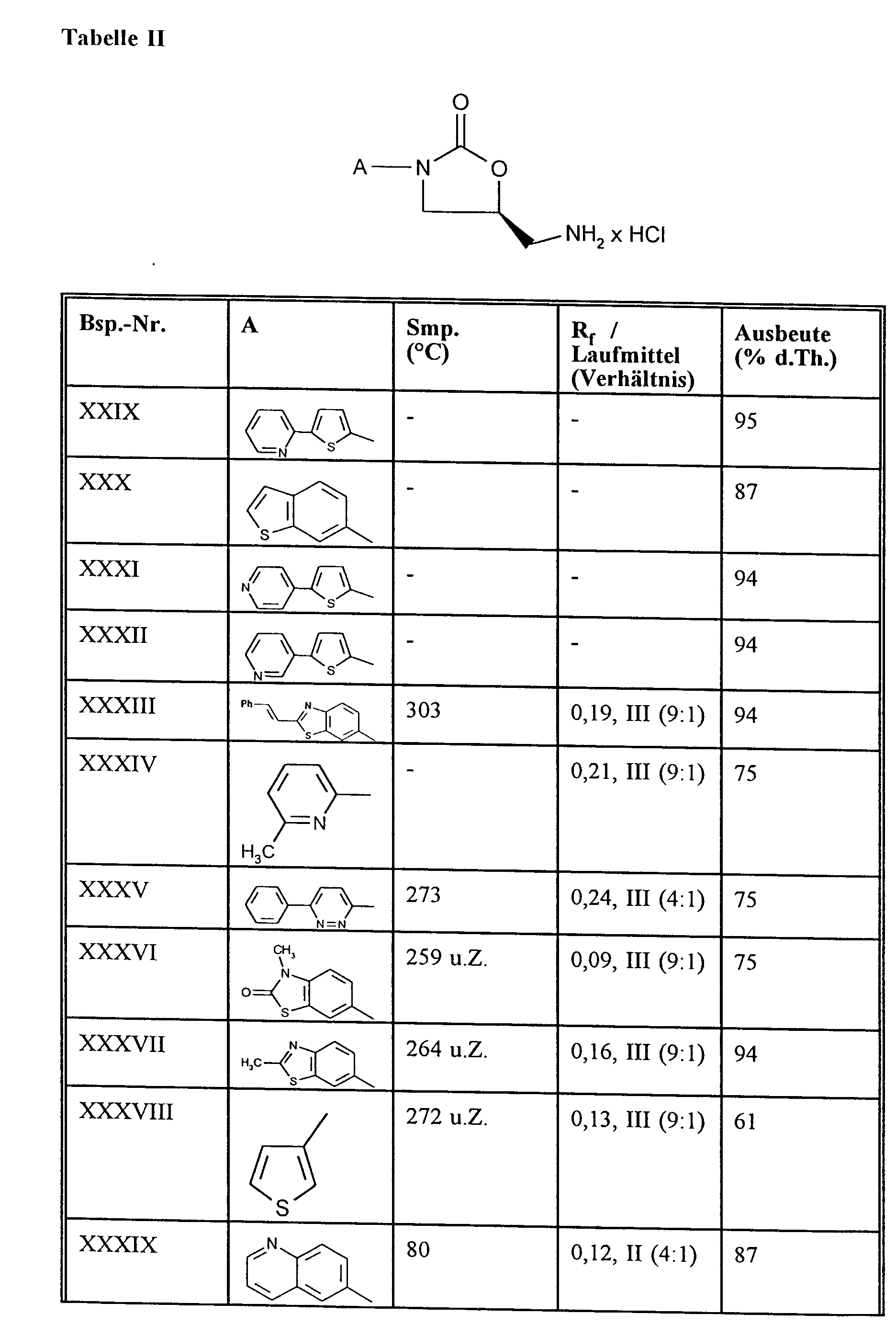

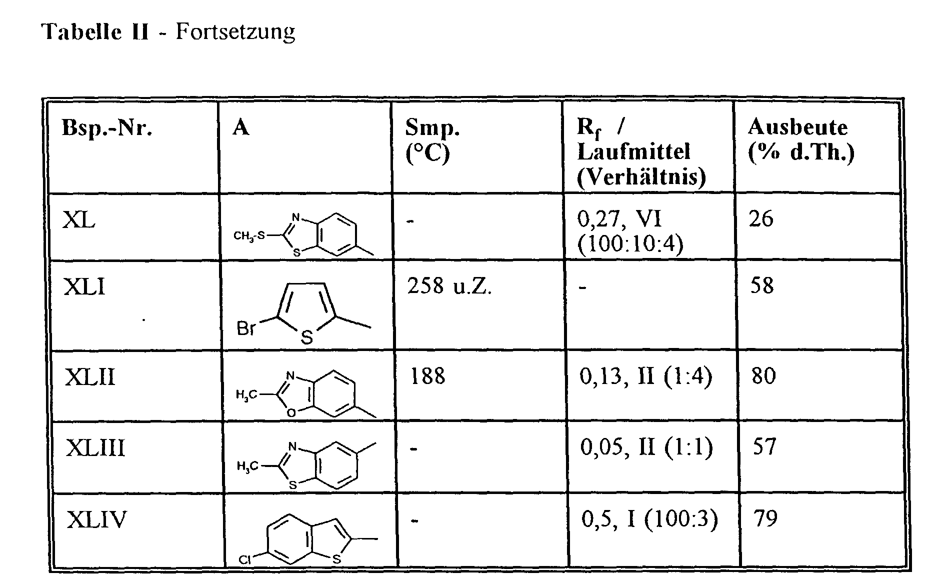

### Beispiel XLV

### (5S)-3-(3-Isopropyl-2-benzoxazolinon-6-yl)-5-aminomethyl-2-oxazolidinon-hydrochlorid

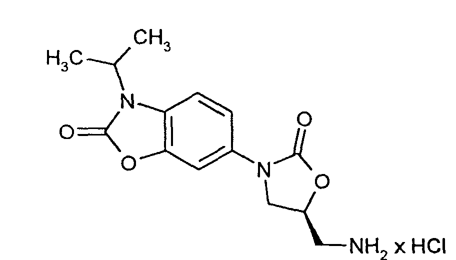

In Analogie zur Vorschrift des Beispiels IV wird aus 3-Benzyloxy-4-nitro-benzoesäure das entsprechende Azid dargestellt (Ausbeute quant.). In Analogie zur Vorschrift des Beispiels V wird aus 3-Benzyloxy-4-nitrobenzoesäureazid das entsprechende Butylcarbamat dargestellt (Ausbeute: 63%, R_{f} (VII, 95:5) = 0,51).

3-Benzyloxy-1-butoxycarbonylamino-4-nitrobenzol wird analog der Vorschrift des Beispiels VI umgesetzt zum entsprechenden Oxazolidinon (Ausbeute: 73%; R_{f} (II, 1:4) = 0,24).

In Analogie zu den Vorschriften der Beispiele XXIII bis XXVIII wird aus (5R)-3-(3-Benzyloxy-4-nitrophenyl)-5-hydroxymethyl-3-oxazolidinon das entsprechende Amin dargestellt (Ausbeuten: 92%, 92% bzw. 83%, R_{f} (VIII, 85:10:5) = 0,08).

Eine Mischung aus (5S)-3-(3-Benzyloxy-4-nitrophenyl)-5-aminomethyl-2-oxazolidinon (20,6 g, 0,06 mol) und Di-tert.-butyldicarbonat (14,4 g, 0,066 mol) in Dichlormethan (300 mL) wird 14 h bei 0°C gerührt. Das Reaktionsgemisch wird eingeengt und das Produkt mit Petrolether gefällt (Ausbeute: 25,4 g (95%), R_{f} (I, 10:1) = 0,80).

(5S)-3-(Benzyloxy-4-nitrophenyl)-5-(tert.-butoxycarbonylaminoethyl)-2-oxazolidinon (25,3 g, 0,057 mol) in Methanol/THF (2:3, 500 mL) wird mit Pd/C (10%, 0,5 g) 14 h unter Wasserstsoff (1 atm) gerührt. Anschließend wird vom Katalysator abfiltriert und die Lösemittel abgezogen (Ausbeute: 18,9 g (quant.), R_{f} (I, 10:1) = 0,28).

Zu einer Mischung aus (5S)-3-(4-Amino-3-hydroxyphenyl)-5-(tert.-butoxycarbonylaminomethyl)-2-oxazolidinon (18,9 g, 0,058 mol), Aceton (8,6 mL, 0,116 mol) und THF (500 mL) wird bei 0°C 1 M Boran-Tetrahydrofuran-Komplex (64 mL, 64 mmol) gegeben und weitere 24 h bei Raumtemperatur gerührt. Die Lösung wird mit 1 M Natriumhydroxid (58 mL, 58 mmol) versetzt, getrocknet (Na₂SO₄) und das Lösemittel im Vakuum abgezogen (Ausbeute: 16,1 g (quant.), R_{f} (I, 10:1) = 0,53).

Eine Lösung von (5S)-3-(4-Isopropylamino-3-hydroxyphenyl)-5-(tert.-butoxycarbonylaminomethyl)-2-oxazolidinon (8,8 g, 24 mmol) und Carbonyldiimidazol (CDI, 4,1 g, 25,2 g) in DMF (200 mL) wird 4 h bei Raumtemperatur gerührt. Der Ansatz wird auf Eiswasser gegeben und der ausgefallene Niederschlag abgesaugt (Ausbeute: 9,0 g (96%), R_{f} (I, 10:1) = 0,71).

Eine Suspension von (5S)-3-(Isopropyl-2-benzothiazolinon-6-yl)-5-(tert.-butoxycarbonylaminomethyl)-2-oxazolidinon (9,0 g, 23 mmol) und Dioxan (200 mL) wird mit 4 M HCl (in Dioxan, 72 mL, 287 mmol) versetzt und 14 h bei Raumtemperatur gerührt. Der ausgefallene Niederschlag wird abgesaugt, mit Ether gewaschen (2 x) und getrocknet.
Ausbeute: 7,4 g (99%)
MS(CI, NH₃): m/z = 309 (M-Cl+NH₄⁺)
¹H-NMR (200 MHz, [D₆]DMSO): δ = 8,5 (bs, 3H, NH₃Cl), 7,62 (d, 1H, Ar-H), 7,45 (d, 1H, Ar-H), 7,25 (dd, 1H, Ar-H), 5,00 (m, 1H, 5-H), 4,45 (m, 1H, HCMe₂), 4,31 (t, 1H, 4-H), 3,90 (dd, 1H, 4-H), 3,20 (d, 2H, CH₂N), 1,45 (d, 6H, CH₃).

### Beispiel XLVI

### (5S)-3-(3-Isopropyl-2-benzyloxazolinon-6-yl)-5-(benzyloxacetylaminomethyl)-2-oxazolidinon

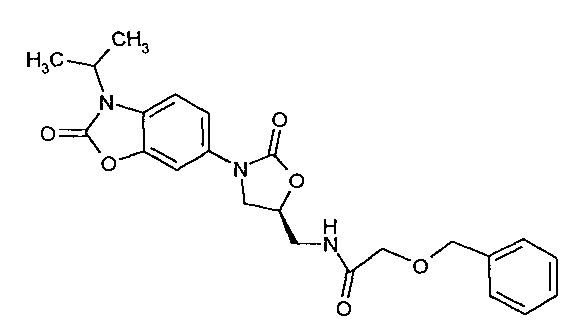

In Analogie zur Vorschrift des Beispiels 3 wird aus dem entsprechenden Hydrochlorid (Beispiel XLV) und Benzyloxyacetylchlorid die Titelverbindung dargestellt.
Ausbeute: 93%
R_{f} (I, 10:1) = 0,69

### Herstellungsbeispiele

### Beispiel 1

### (5S)-3-(5-[3-Pyridyl]-pyridin-2-yl)-5-(2-nitro-prop-1-en-1-yl-aminomethyl)oxazolidin-2-on

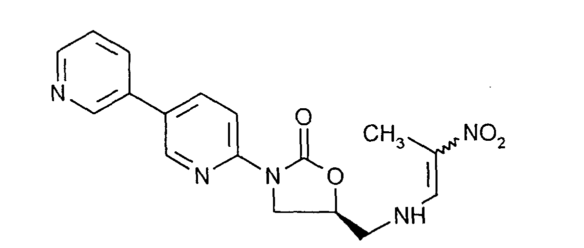

Unter Argon löst man 100 mg (0,37 mmol) der Verbindung aus Beispiel XXVIII (freie Base; hergestellt durch Lösen in Wasser, NH_{3(aq)}-Zugabe bis pH 11, Extraktion mit CH₂Cl₂, Trocknen über MgSO₄ und Einengen) in 1 ml DMF und gibt 200 mg (1,11 mmol) 2-(2-Nitro-prop-1-en-1-yl-amino)-pyridin zu und läßt über Nacht rühren. Man versetzt mit Wasser, extrahiert 3 x mit Essigester, wäscht die organische Phase mit ges. NaCl-Lösung und trocknet über MgSO₄. Man engt ein und reinigt durch Säulenchromatographie an Kieselgel (Laufmittel CH₂Cl₂ : MeOH = 100:5). Man erhält 126 mg (96% d.Th.) der Titelverbindung.
Fp.: 207°C
R_{f}-Wert: (CH₂Cl₂ : MeOH = 10:1) 0,57
MS (DCI): 356 (M+H)⁺
¹H-NMR (200 MHz, DMSO-d₆): δ = 8,95 (d, J = 2, 1H); 8,79 (d, J = 2, 1H); 8,60 (dd, J = 5, J = 2, 1H); 8,4 - 7,9 und 7,6 - 7,4 (m, insgesamt 6H); 4,9 (m, 1H); 4,3 (m, 1H); 4,05 (m, 1H); 3,7 (m, 2H); 1,95 und 1,93 (s, insgesamt 3H).

### Beispiel 2

### (5R)-3-(2-Methyl-benzo[4,5-d]thiazol-6-yl)-5-(2-thiazolyl-aminomethyl)-oxazolidin-2-on

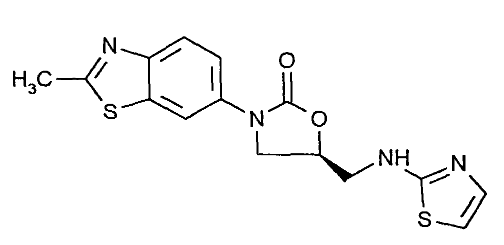

Unter Argon werden 292 mg (2,92 mmol) 2-Aminothiazol in 5 ml abs. THF vorgelegt und bei -78°C mit 1,33 ml (2,92 mmol) 2,2 M n-BuLi-Lösung versetzt. Man läßt 30 Minuten bei -78°C rühren. Man addiert 0,5 g (1,46 mmol) (5R)-3-(2-Methyl-benzo[4,5-d]thiazol-6-yl)-5-methoxysulfonyloxymethyl-oxazolidin-2-on, gelöst in 5 ml abs. THF, und läßt 1 h bei -78°C rühren. Man entfernt das Kühlbad und läßt über Nacht rühren. Man addiert NH₄Cl-Lösung und HCl-Lösung (auf pH 3), extrahiert mit Chloroform, trocknet über MgSO₄ und engt ein. Die Substanz wird säulenchromatographisch an Kieselgel gereinigt (Laufmittel CH₂Cl₂ : MeOH = 100:1 bis 100:3). Man erhält 193 mg (38% d.Th.) der Titelverbindung.
Fp.: 207°C
R_{f}= 0,47 (CH₂Cl₂ : MeOH = 10:1)

### Beispiel 3

### (5R)-3-(5-(2-Pyridyl)-thien-2-yl)-5-thioacetylamino-methyl-oxazolidin-2-on

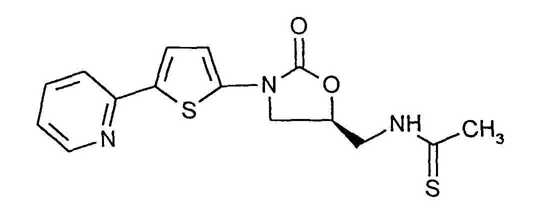

3,48 mg (1 mmol) der Verbindung aus Beispiel XXIX werden mit 4 ml THF und 0,24 ml (1,7 mmol) Triethylamin versetzt. Zu der so erhaltenen Reaktionsmischung gibt man unter Rühren 152 µl (1,1 mmol) Dithioessigsäureethylester und hält 24 h bei Raumtemperatur. Nach Einengen wird mit Methylenchlorid / Methanol (100:2) an Kieselgel chromatographiert.
Ausbeute: 100 mg (36% d.Th.)
Smp.: 181°C u.Z.
R_{f} : 0,36 (I; 100:5)
¹H-NMR (D₆-DMSO, 300 MHz): δ = 10,37 (br, 1H); 8,47 (d, J = 5 Hz, 1H); 7,75 - 7,88 (m, 2H); 7,62 (d, J = 5 Hz, 1H); 7,2 (m, 1H); 6,58 (d, J = 5 Hz, 1H); 5,03 - 5,13 (m, 1H); 4,21 (dd, J = 10 Hz, 9 Hz, 1H); 3,95 (t, J = 6 Hz, 2H); 3,85 (dd, J = 10 Hz, 6 HZ, 1H); 2,45 (s, 3H).

Analog den Vorschriften der Beispiele 1 bis 3 werden die in Tabelle 1 aufgeführten Verbindungen dargestellt:

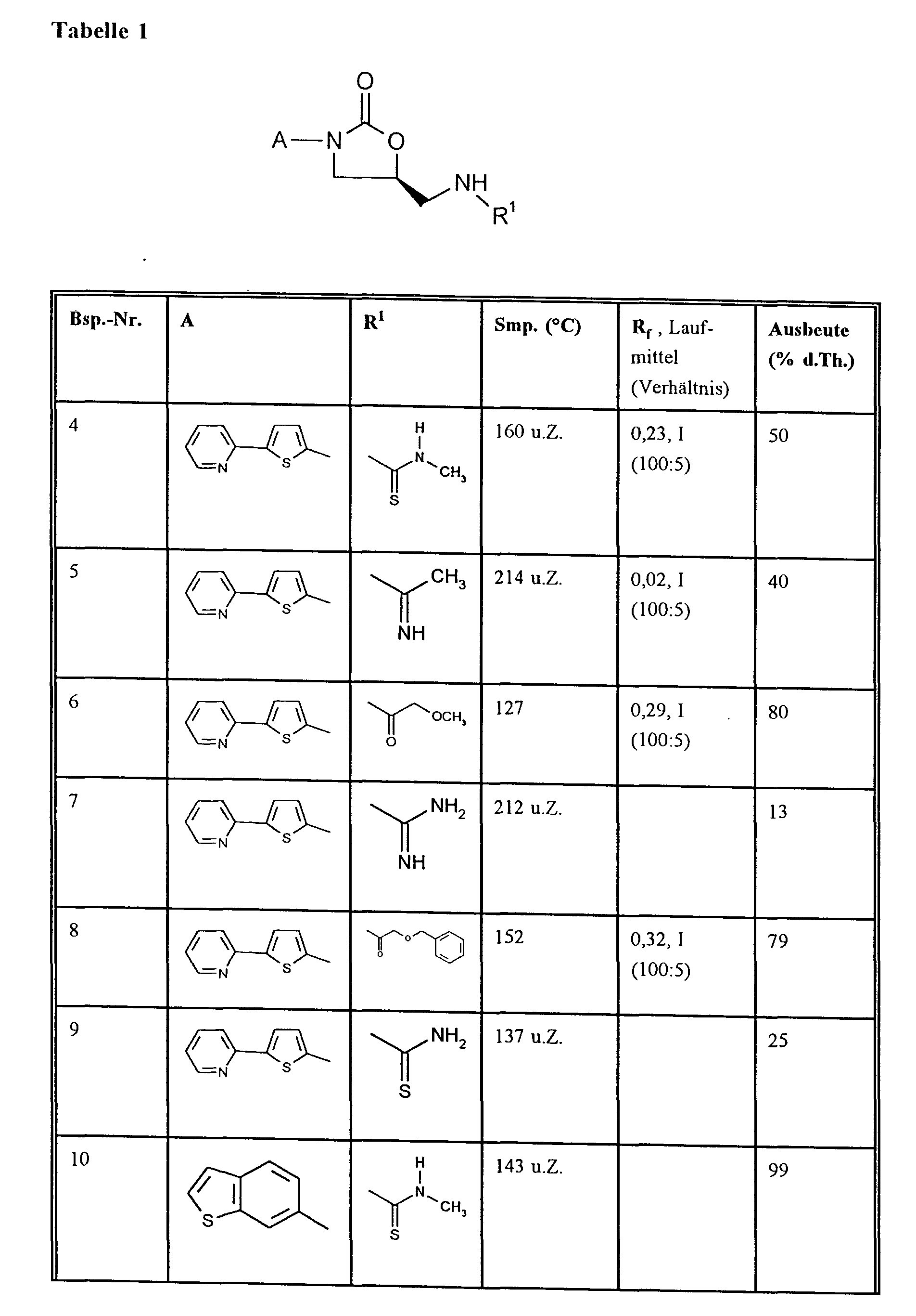

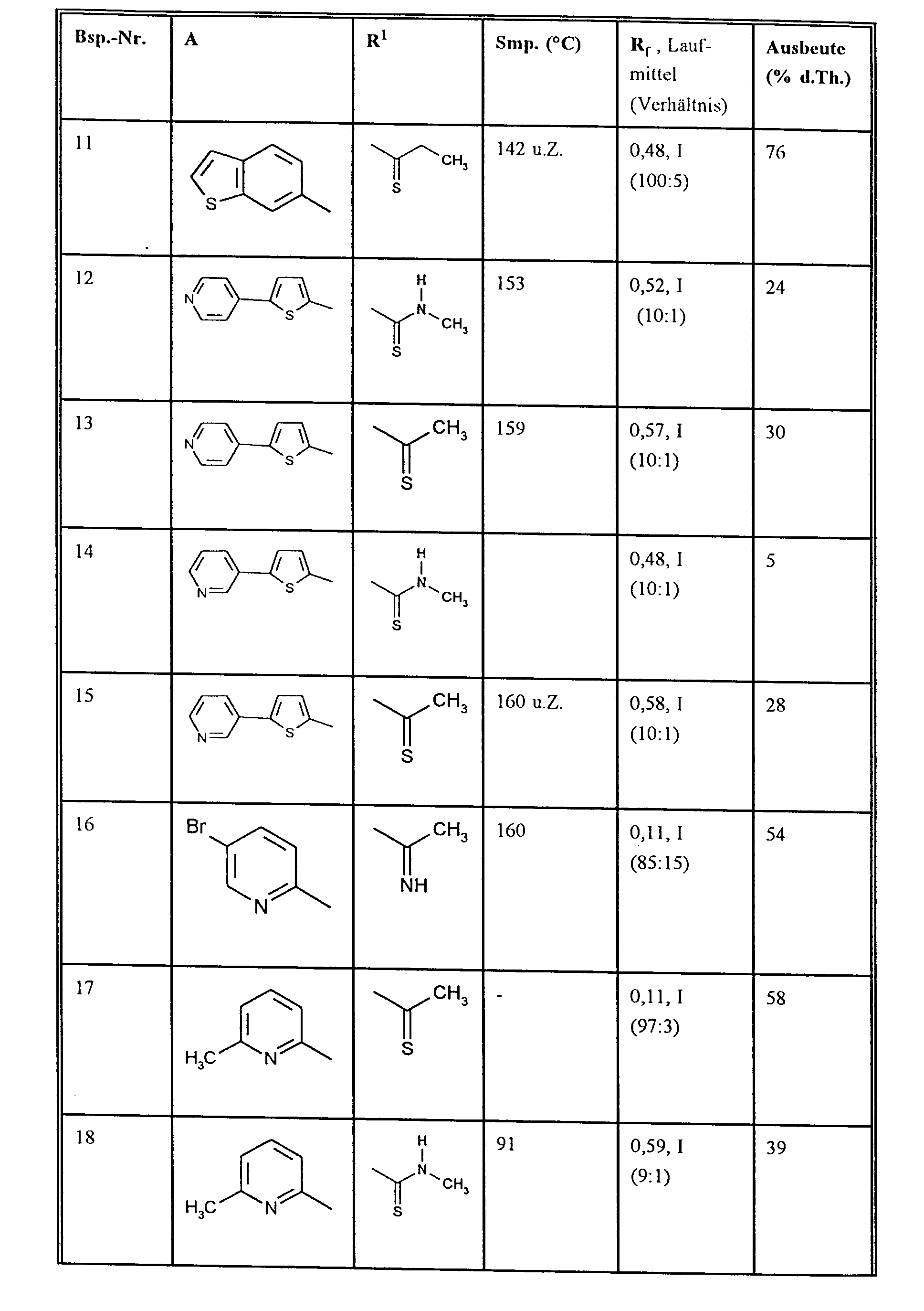

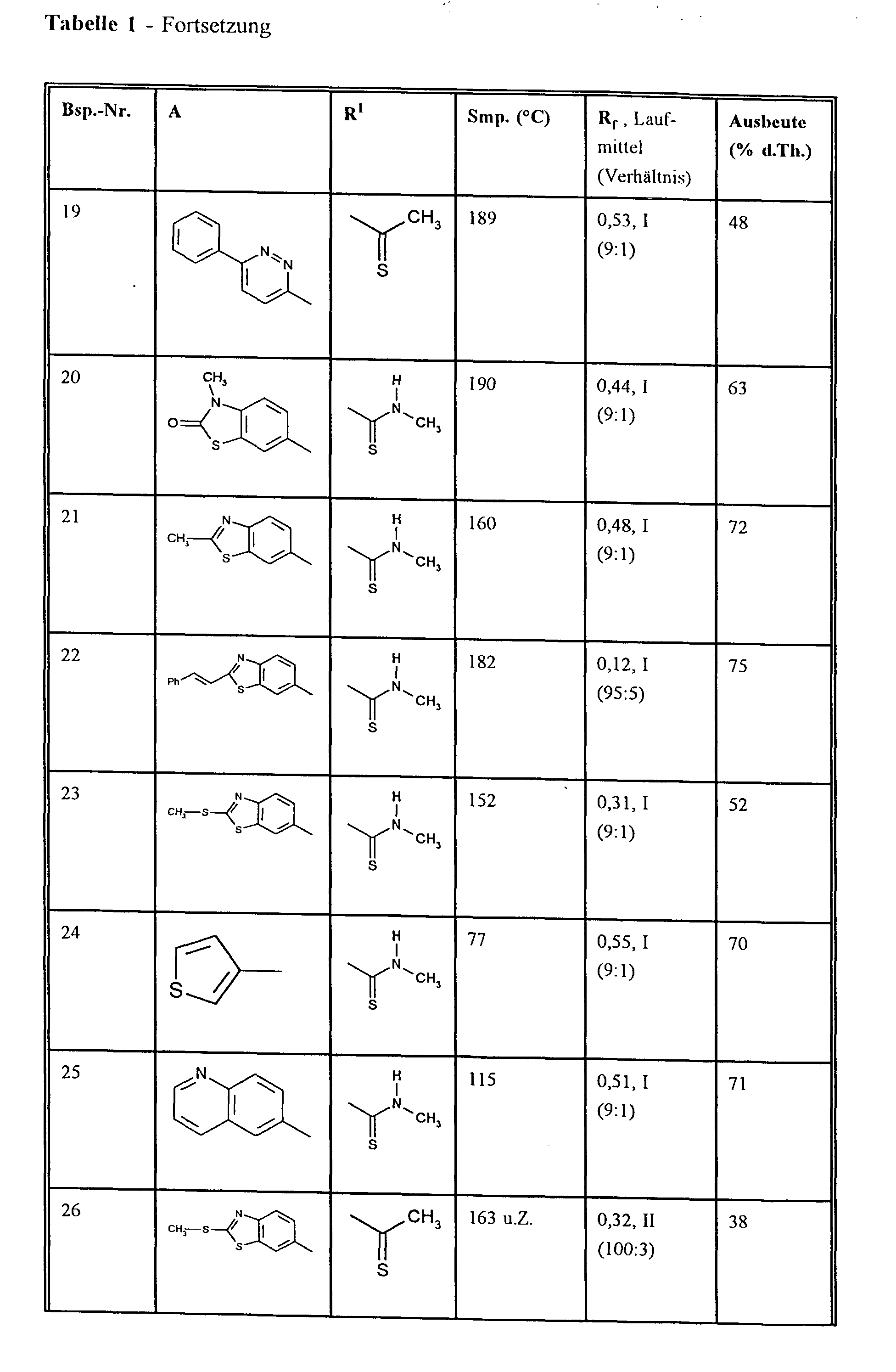

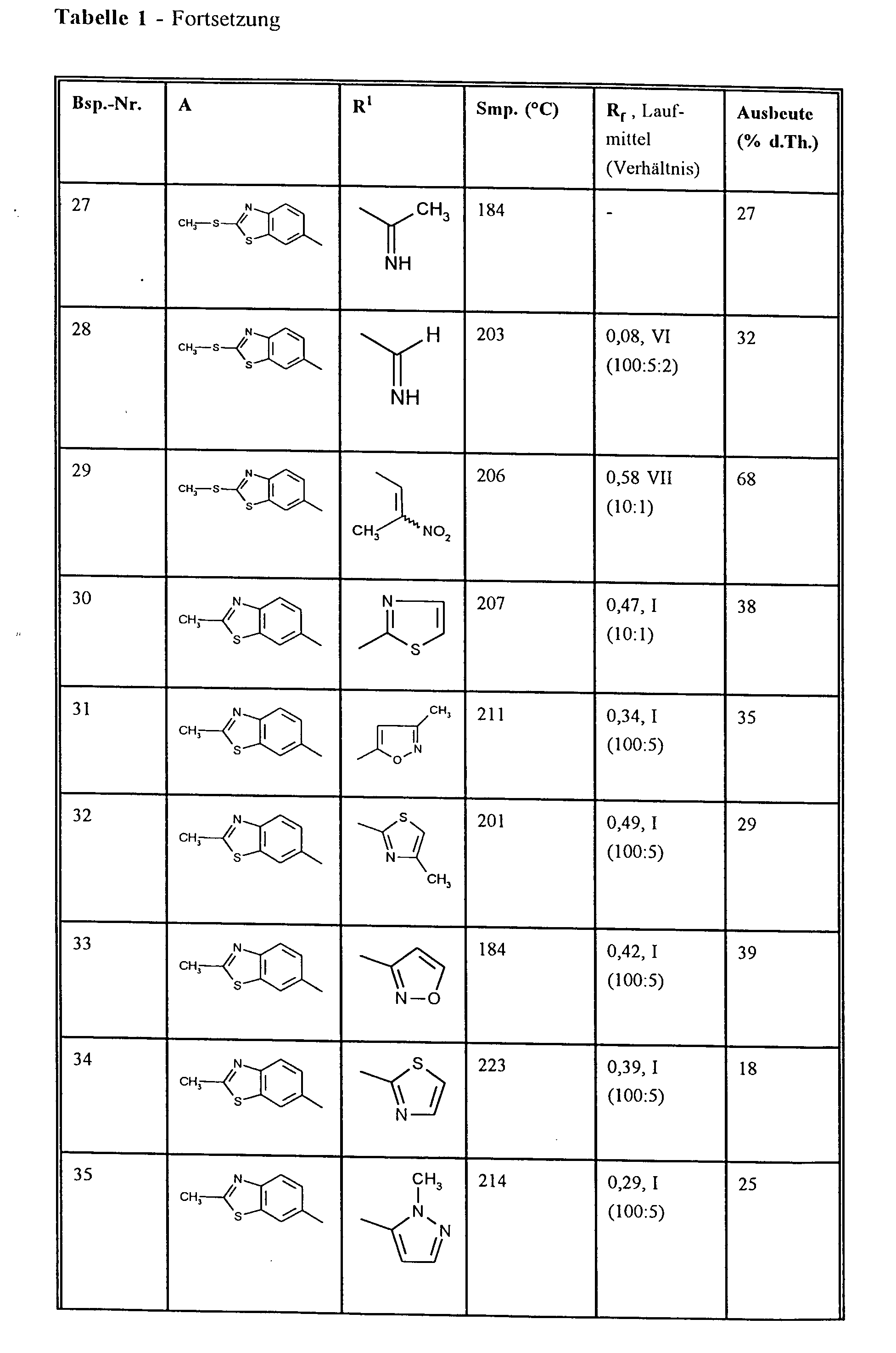

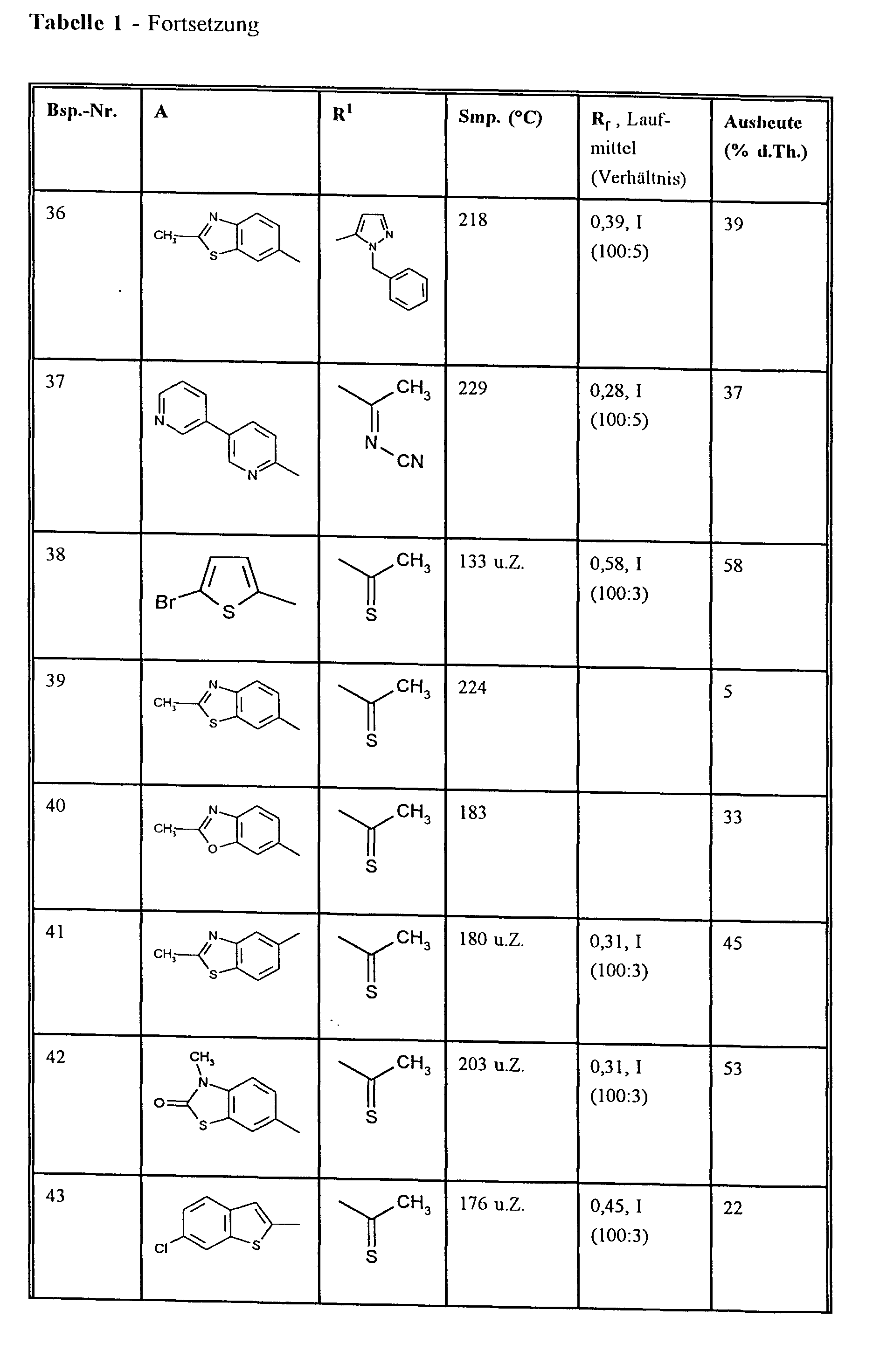

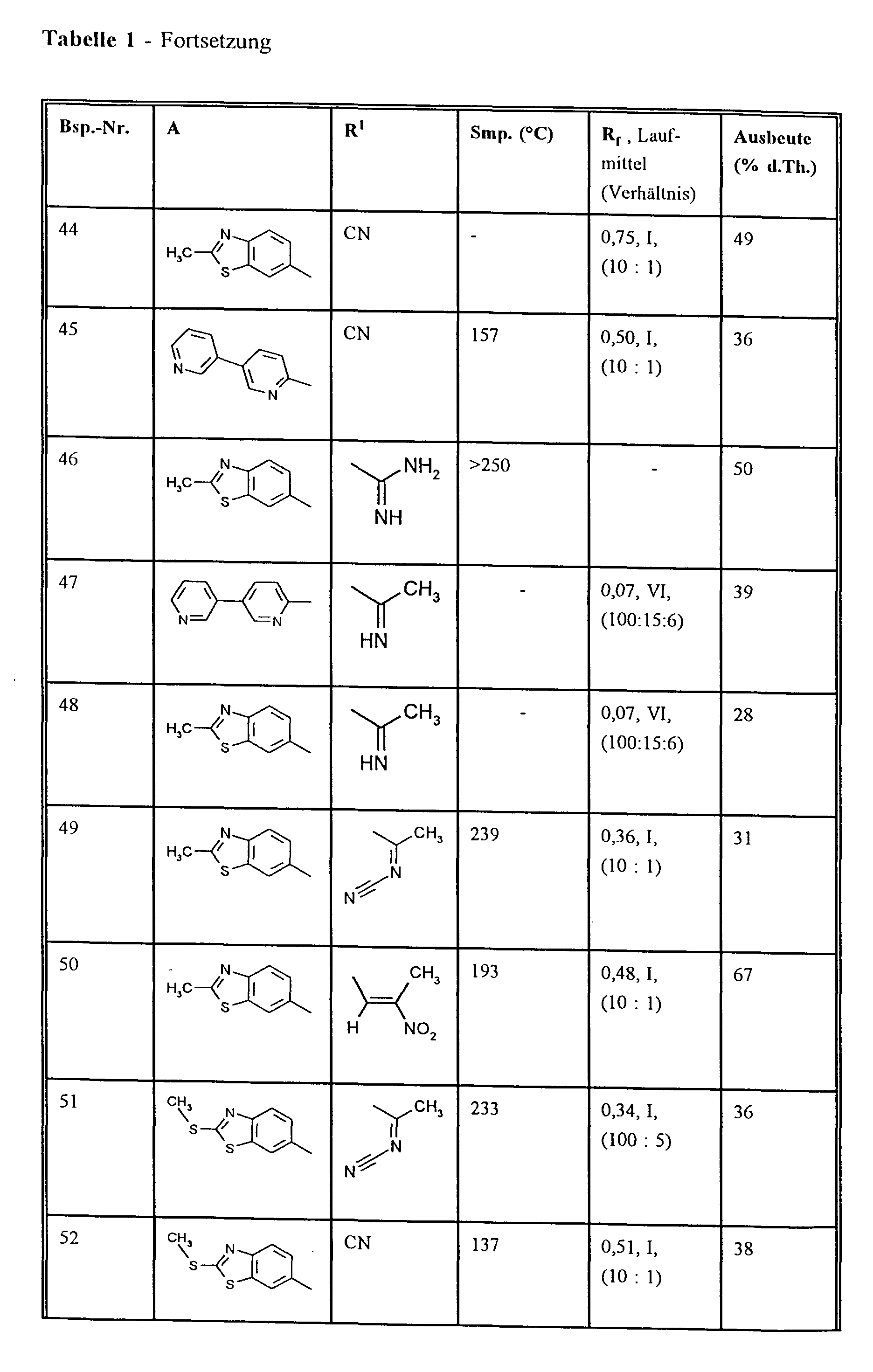

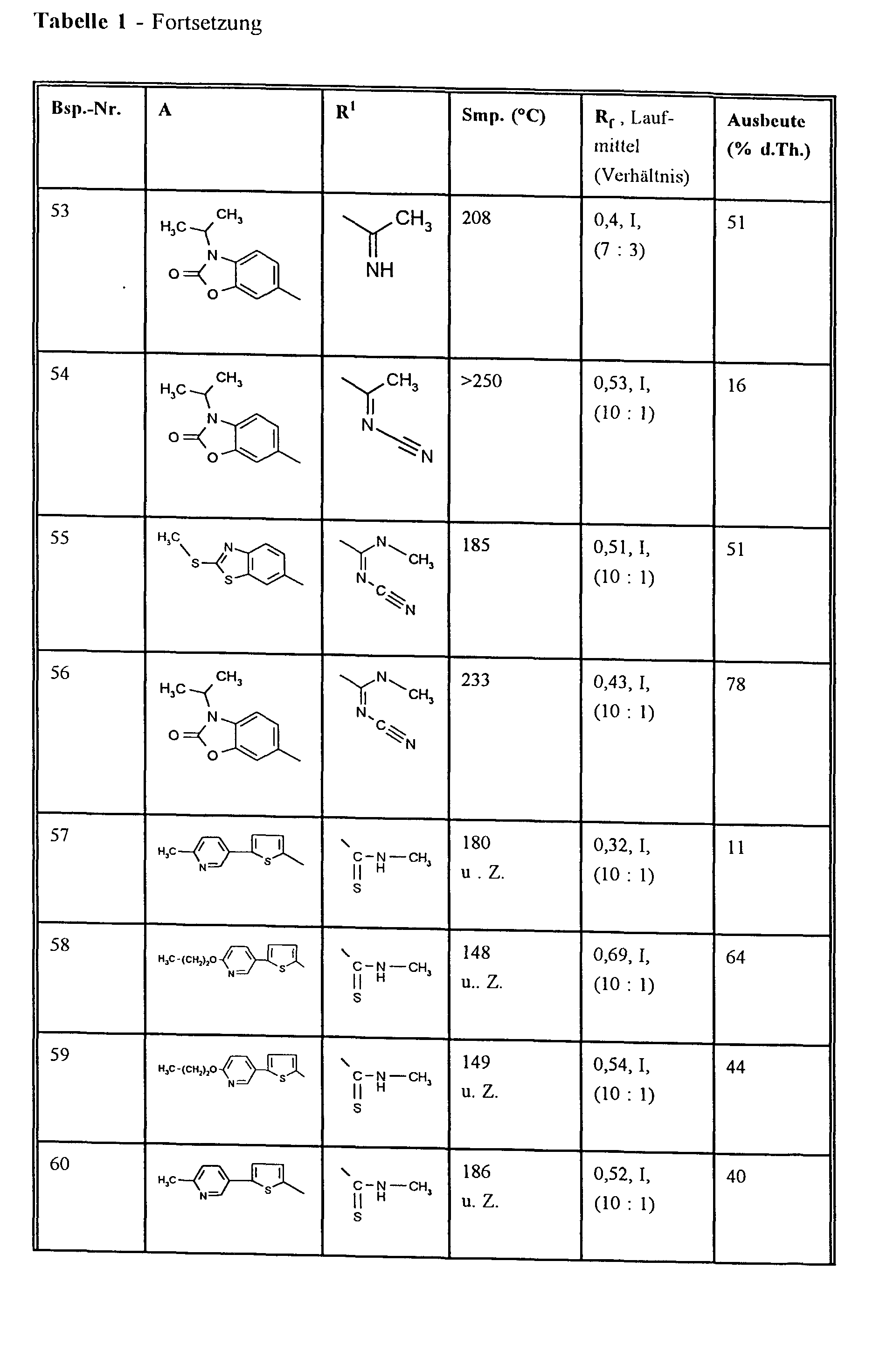

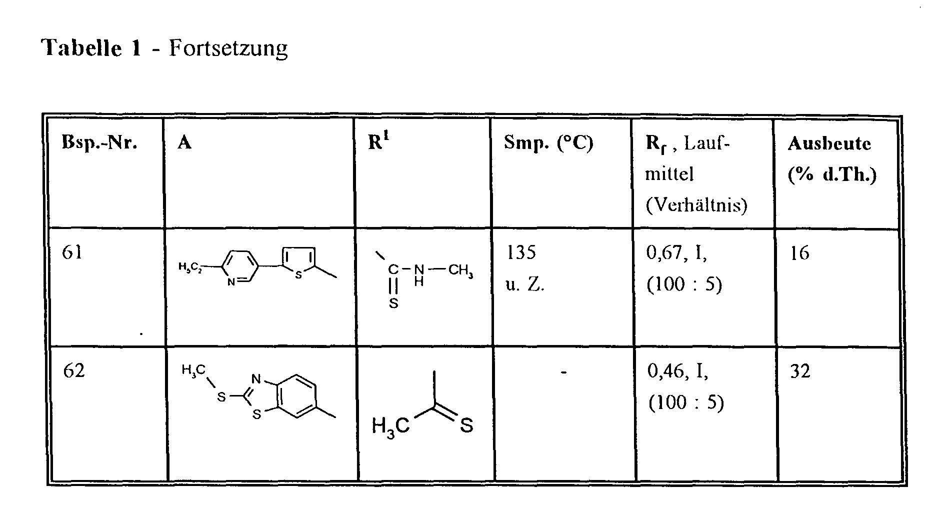

### Beispiel 63

### (5S)-3-(3-Isopropyl-2-benzoxazolinon-6-yl)-5-hydroxyacetylamino-methyl-2-oxazolidinon

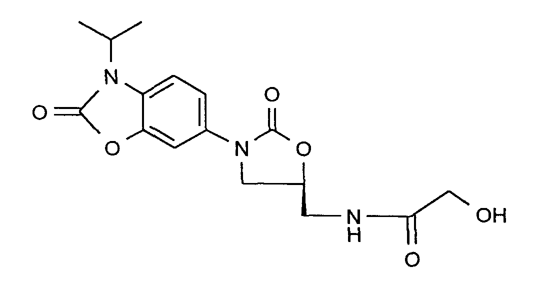

Die Verbindung aus Beispiel XLVI (390 mg, 0,89 mmol) in THF (10 ml) und Essigester (10 ml) wird mit Pd(OH)₂/C (5 %, 30 mg) unter 3 bar Wasserstoff gerührt. Es wird vom Katalysator abfiltriert, der Rückstand mit THF gewaschen, das Filtrat eingeengt und mit Dichlormethan verrührt. Man erhält 36 mg (11 %) der Titelverbindung als weißen Feststoff.
Ausbeute: 11 %
R_{f}-Wert: (I, 10:1) 0,23
MS (CI): 367 (M+NH₄⁺)
¹H-NMR (200 MHz, [D₆]DMSO): δ = 8,05 (bt, 1H), 7,62 (dd, 1H), 7,43 (d, 1H), 7,23 (dd, 1H), 5,60 (t, 1H, OH), 4,75 (m, 1H), 4,50 (m, 1H), 4,20 (t, 1H), 3,80 (m, 3H), 3,50 (m, 2H), 1,50 (d, 6H).

## Patentansprüche

1. Substituierte Oxazolidinone der allgemeinen Formel (I) 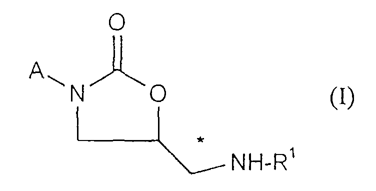 in welcher
A für einen über ein Kohlenstoffatom direkt gebundenen 5-gliedrigen aromatischen Heterocyclus mit bis zu 3 Heteroatomen aus der Reihe S, N und/oder O steht, der zusätzlich einen annellierten Benzol- oder Naphthylring besitzen kann, oder
für einen über ein Kohlenstoffatom direkt gebundenen 6-gliedrigen, aromatischen Heterocyclus mit mindestens einem Stickstoffatom steht, oder
für einen über ein Kohlenstoffatom direkt gebundenen, jeweils 6-gliedrigen, bi- oder tricyclischen aromatischen Rest mit mindestens einem stickstoffhaltigen Ring steht, oder
für ß-Carbolin-3-yl oder für über den 6-Ring direkt gebundenes Indolizinyl steht, wobei die oben genannten Cyclen gegebenenfalls jeweils bis zu 3-fach gleich oder verschieden durch Carboxy, Halogen, Cyano,
Mercapto, Formyl, Phenyl, Trifluormethyl, Nitro, geradkettiges oder verzweigtes Alkoxy, Alkoxycarbonyl, Alkylthio oder Acyl mit jeweils bis zu 6 Kohlenstoffatomen oder durch geradkettiges oder verzweigtes Alkyl oder Alkenyl mit jeweils bis zu 6 Kohlenstoffatomen substituiert sind, die ihrerseits durch Phenyl substituiert sein können, und/oder durch Pyridyl substituiert sind, das seinerseits durch geradkettiges Alkyl oder Alkoxy mit jeweils bis zu 6 Kohlenstoffatomen substituiert sein kann oder
für einen Rest der Formel 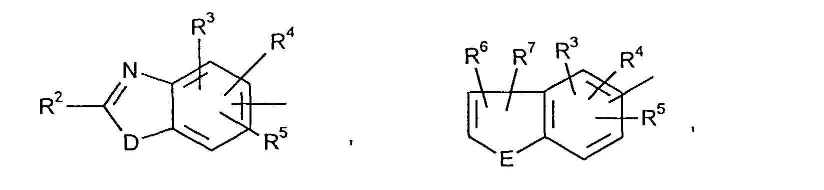 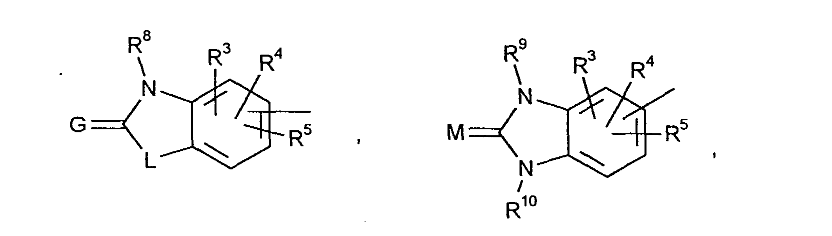 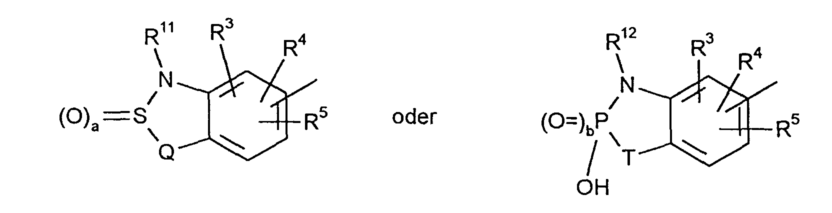 steht,
worin
R³, R⁴, R⁵, R⁶ und R⁷ gleich oder verschieden sind und Wasserstoff oder Carboxy, Halogen, Cyano, Formyl, Trifluormethyl, Nitro, geradkettiges oder verzweigtes Alkyl mit bis zu 6 Kohlenstoffatomen oder eine Gruppe der Formel - CO-NR¹³R¹⁴ bedeuten,
worin
R¹³ und R¹⁴ gleich oder verschieden sind und Wasserstoff, geradkettiges oder verzweigtes Alkyl mit bis zu 4 Kohlenstoffatomen oder Phenyl bedeuten,
R², R⁸, R⁹, R¹⁰, R¹¹ und R¹² gleich oder verschieden sind und Wasserstoff, Cycloalkylcarbonyl oder Cycloalkyl mit jeweils 3 bis 6 Kohlenstoffatomen, oder geradkettiges oder verzweigtes Alkoxycarbonyl oder Alkylthio mit jeweils bis zu 6 Kohlenstoffatomen bedeuten, oder geradkettiges oder verzweigtes Alkyl mit bis zu 10 Kohlenstoffatomen bedeuten, das gegebenenfalls durch Cyano, Trifluormethyl, Halogen, Phenyl, Hydroxy, Carboxyl, geradkettiges oder verzweigtes Alkoxycarbonyl mit bis zu 6 Kohlenstoffatomen, Aryl mit 6 bis 10 Kohlenstoffatomen, Cycloalkyl mit 3 bis 6 Kohlenstoffatomen und/oder durch eine Gruppe der Formel -(CO)_{c}-NR¹⁵R¹⁶, R¹⁷-N-SO₂-R¹⁸, R¹⁹R²⁰-N-SO₂- oder R²¹-S(O)_{d} substituiert ist,
worin
c eine Zahl 0 oder 1 bedeutet,
R¹⁵, R¹⁶ und R¹⁷ die oben angegebene Bedeutung von R¹³ und R¹⁴ haben und mit dieser gleich oder verschieden sind,
oder gemeinsam mit dem Stickstoffatom einen 5- bis 6-gliedrigen, gesättigten Heterocyclus mit gegebenenfalls einem weiteren Heteroatom aus der Serie N, S und/oder O bilden, der seinerseits gegebenenfalls, auch an einem weiteren Stickstoffatom, durch geradkettiges oder verzweigtes Alkyl oder Acyl mit bis zu 3 Kohlenstoffatomen substituiert sein kann,
R¹⁹ und R²⁰ die oben angegebene Bedeutung von R¹³ und R¹⁴ haben und mit dieser gleich oder verschieden sind,
d eine Zahl 0, 1 oder 2 bedeutet,
R¹⁸ und R²¹ gleich oder verschieden sind und geradkettiges oder verzweigtes Alkyl mit bis zu 4 Kohlenstoffatomen, Benzyl, Phenyl oder Tolyl bedeuten,
oder
geradkettiges oder verzweigtes Acyl mit bis zu 6 Kohlenstoffatomen bedeuten, das gegebenenfalls durch Trifluormethyl, Trichlormethyl oder durch eine Gruppe der Formel -OR²² substituiert ist,
worin
R²² Wasserstoff oder geradkettiges oder verzweigtes Alkyl mit bis zu 6 Kohlenstoffatomen bedeutet, das gegebenenfalls durch Aryl mit bis zu 10 Kohlenstoffatomen substituiert ist,
oder
eine Gruppe der Formel -(CO)ₑ-NR²³R²⁴, -NR²⁵-SO₂R²⁶, R²⁷R²⁸-NSO₂- oder R²⁹-S(O)_{f} bedeuten,
worin
e die oben angegebene Bedeutung von c hat und mit dieser gleich oder verschieden ist,
R²³ und R²⁴ und R²⁵ jeweils die oben angegebene Bedeutung von R¹⁵, R¹⁶ und R¹⁷ haben und mit dieser gleich oder verschieden sind,
R²⁷ und R²⁸ die oben angegebene Bedeutung von R¹³ und R¹⁴ haben und mit dieser gleich oder verschieden sind,
f die oben angegebene Bedeutung von d hat und mit dieser gleich oder verschieden ist,
R²⁶ und R²⁹ die jeweils oben angegebene Bedeutung von R¹⁸ und R²¹ haben und mit dieser gleich oder verschieden sind,
D ein Sauerstoffatom oder einen Rest der Formel -S(O)_{g} bedeutet,
worin
g eine Zahl 0, 1 oder 2 bedeutet,
E und L gleich oder verschieden sind und ein Sauerstoff- oder ein Schwefelatom bedeuten,
G, M, T und Q gleich oder verschieden sind und ein Sauerstoff- oder ein Schwefelatom, oder eine Gruppe der Formel -NR³⁰ bedeuten,
worin
R³⁰ Wasserstoff oder geradkettiges oder verzweigtes Alkyl mit bis zu 5 Kohlenstoffatomen bedeutet,
a und b gleich oder verschieden sind und eine Zahl 1 oder 2 bedeuten,
R¹ für einen Rest der Formel 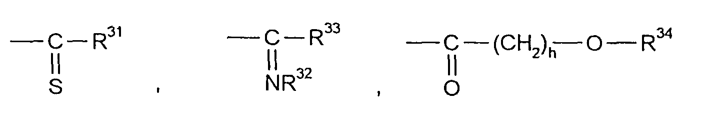 oder 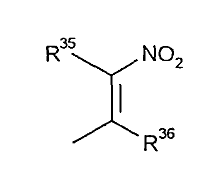 steht,
worin
R³¹ geradkettiges oder verzweigtes Alkyl mit bis zu 7 Kohlenstoffatomen, Cycloalkyl mit 3 bis 6 Kohlenstoffatomen, Phenyl oder eine Gruppe der Formel -NR³⁸R³⁹ bedeutet,
worin
R³⁸ und R³⁹ die oben angegebene Bedeutung von R¹³ und R¹⁴ haben und mit dieser gleich oder verschieden sind,
R³² Wasserstoff, Cyano, Cycloalkyl mit 3 bis 6 Kohlenstoffatomen, Phenyl oder geradkettiges oder verzweigtes Alkyl mit bis zu 7 Kohlenstoffatomen bedeutet,
R³³ Wasserstoff, geradkettiges oder verzweigtes Alkyl mit bis zu 7 Kohlenstoffatomen, Phenyl, Cycloalkyl mit 3 bis 6 Kohlenstoffatomen oder eine Gruppe der Formel -NR⁴⁰R⁴¹ bedeutet,
worin
R⁴⁰ und R⁴¹ die oben angegebene Bedeutung von R¹³ und R¹⁴ haben und mit dieser gleich oder verschieden sind,
h eine Zahl 1, 2, 3 oder 4 bedeutet,
R³⁴ Wasserstoff, geradkettiges oder verzweigtes Alkyl mit bis zu 6 Kohlenstoffatomen oder Benzyl bedeutet,
R³⁵ und R³⁶ gleich oder verschieden sind und Wassserstoff oder geradkettiges oder verzweigtes Alkyl mit bis zu 6 Kohlenstoffatomen bedeuten,
oder
R¹ für Cyano oder für einen 5- bis 7-gliedrigen, gesättigten, partiell ungesättigten oder ungesättigten Heterocyclus mit bis zu 3 Heteroatomen aus der Reihe S, N und/oder O steht, der gegebenenfalls auch über eine N-Funktion, bis zu 2-fach gleich oder verschieden durch Benzyl, Halogen oder durch geradkettiges oder verzweigtes Alkyl mit bis zu 5 Kohlenstoffatomen substituiert ist,
und deren Stereoisomere, Stereoisomerengemische und Salze.

2. Verbindungen der allgemeinen Formel (I) gemäß Anspruch 1,
in welcher
A für jeweils über ein Kohlenstoffatom gebundenes Chinolyl, Benzothiophenyl, Benzthiazolyl, Benzoxazolyl, Pyridyl, Pyridazyl oder Thienyl steht, die gegebenenfalls bis zu 3-fach gleich oder verschieden durch Fluor, Chlor, Brom, Phenyl oder durch geradkettiges oder verzweigtes Alkyl oder Alkylthio mit jeweils bis zu 4 Kohlenstoffatomen oder durch geradkettiges oder verzweigtes Alkenyl mit bis zu 4 Kohlenstoffatomen substituiert sind, das seinerseits durch Phenyl substituiert sein kann, und/oder durch Pyridyl substituiert sind, das seinerseits durch geradkettiges oder verzweigtes Alkyl oder Alkoxy mit jeweils bis zu 5 Kohlenstoffatomen substituiert sein kann, oder für einen Rest der Formel 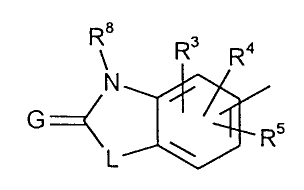 steht,
worin
G ein Sauerstoff- oder Schwefelatom bedeutet,
L ein Sauerstoff- oder Schwefelatom bedeuet,
R⁸ geradkettiges oder verzweigtes Alkyl oder Alkylthio mit jeweils bis zu 6 Kohlenstoffatomen bedeutet,
R³, R⁴ und R⁵ gleich oder verschieden sind und
Wasserstoff, Fluor, Chlor oder Brom bedeuten,
R¹ für einen Rest der Formel 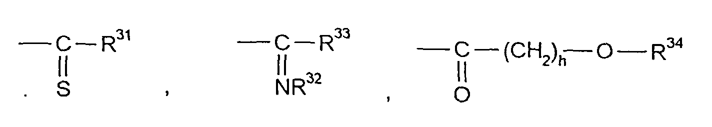 oder 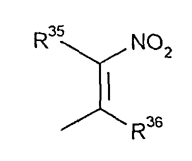 steht,
worin
R³¹ geradkettiges oder verzweigtes Alkyl mit bis zu 5 Kohlenstoffatomen, Cyclopropyl, Cyclobutyl, Cyclopentyl, Cyclohexyl, Phenyl oder eine Gruppe der Formel -NR³⁸R³⁹ bedeutet,
worin
R³⁸ und R³⁹ gleich oder verschieden sind und
Wasserstoff, Methyl oder Ethyl bedeuten,
R³² Wasserstoff, Cyano, Cyclopropyl, Cyclobutyl, Cyclopentyl, Cyclohexyl, Phenyl oder geradkettiges oder verzweigtes Alkyl mit bis zu 5 Kohlenstoffatomen bedeutet,
R³³ Wasserstoff, geradkettiges oder verzweigtes Alkyl mit bis zu 5 Kohlenstoffatomen, Phenyl, Cyclopropyl, Cyclobutyl, Cyclopentyl, Cyclohexyl oder eine Gruppe der Formel - NR⁴⁰R⁴¹ bedeutet,
worin
R⁴⁰ und R⁴¹ die oben angegebene Bedeutung von R³⁸ und R³⁹ haben und mit dieser gleich oder verschieden sind,
h eine Zahl 1, 2, 3 oder 4 bedeutet,
R³⁴ Wasserstoff, geradkettiges oder verzweigtes Alkyl mit bis zu 4 Kohlenstoffatomen oder Benzyl bedeutet,
R³⁵ und R³⁶ gleich oder verschieden sind und Wassserstoff oder geradkettiges oder verzweigtes Alkyl mit bis zu 4 Kohlenstoffatomen bedeuten,
oder
R¹ für Cyano oder für Thienyl, Oxazolyl, Thiazolyl, Isoxazolyl oder Pyrazolyl steht, die gegebenenfalls, auch über eine N-Funktion, bis zu 2-fach gleich oder verschieden durch Benzyl, Fluor, Chlor, Brom oder durch geradkettiges oder verzweigtes Alkyl mit bis zu 3 Kohlenstoffatomen substituiert sind,
und deren Stereoisomere, Stereoisomerengemische und Salze.

3. Verbindungen der allgemeinen Formel (I) gemäß Anspruch 1,
in welcher
A für jeweils über ein Kohlenstoffatom gebundenes Chinolyl, Benzothiophenyl, Benzthiazolyl, Benzoxazolyl, Pyridyl, Pyridazyl oder Thienyl steht, die gegebenenfalls bis zu 2-fach gleich oder verschieden durch Fluor, Chlor, Brom, Phenyl durch geradkettiges oder verzweigtes Alkyl oder Alkylthio mit jeweils bis zu 3 Kohlenstoffatomen oder durch geradkettiges oder verzweigtes Alkenyl mit bis zu 3 Kohlenstoffatomen substituiert sind, das seinerseits durch Phenyl substituiert sein kann, und/oder durch Pyridyl substituiert sind, das seinerseits durch geradkettiges oder verzweigtes Alkyl oder Alkoxy mit jeweils bis zu 4 Kohlenstoffatomen substituiert sein kann, oder
für einen Rest der Formel 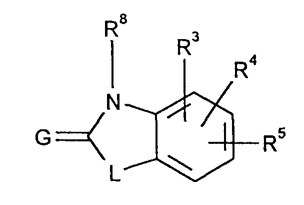 steht,
worin
G ein Sauerstoff- oder Schwefelatom bedeutet,
L ein Sauerstoff- oder Schwefelatom bedeuet,
R⁸ geradkettiges oder verzweigtes Alkyl mit bis zu 4 Kohlenstoffatomen bedeutet,
R³, R⁴ und R⁵ gleich oder verschieden sind und Wasserstoff, Fluor, Chlor oder Brom bedeuten,
R¹ für einen Rest der Formel 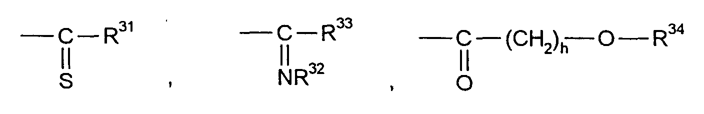 oder 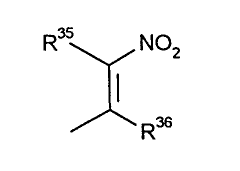 steht,
worin
R³¹ geradkettiges oder verzweigtes Alkyl mit bis zu 4 Kohlenstoffatomen, Cyclopropyl, Cyclobutyl, Cyclopentyl, Cyclohexyl, Phenyl oder eine Gruppe der Formel -NR³⁸R³⁹ bedeutet,
worin
R³⁸ und R³⁹ gleich oder verschieden sind und Wasserstoff oder Methyl bedeuten,
R³² Wasserstoff, Cyano, Cyclopropyl, Cyclobutyl, Cyclopentyl, Cyclohexyl, Phenyl oder geradkettiges oder verzweigtes Alkyl mit bis zu 4 Kohlenstoffatomen bedeutet,
R³³ Wasserstoff, geradkettiges oder verzweigtes Alkyl mit bis zu 4 Kohlenstoffatomen, Phenyl, Cyclopropyl, Cyclobutyl, Cyclopentyl, Cyclohexyl oder eine Gruppe der Formel - NR⁴⁰R⁴¹ bedeutet,
worin
R⁴⁰ und R⁴¹ die oben angegebene Bedeutung von R³⁸ und R³⁹ haben und mit dieser gleich oder verschieden sind,
h eine Zahl 1, 2, 3 oder 4 bedeutet,
R³⁴ Wasserstoff, geradkettiges oder verzweigtes Alkyl mit bis zu 3 Kohlenstoffatomen oder Benzyl bedeutet,
R³⁵ und R³⁶ gleich oder verschieden sind und Wassserstoff oder geradkettiges oder verzweigtes Alkyl mit bis zu 3 Kohlenstoffatomen bedeuten,
oder
R¹ für Cyano oder für Thienyl, Thiazolyl, Isoxazolyl oder Pyrazolyl steht, die gegebenenfalls auch über eine N-Funktion bis zu 2-fach gleich oder verschieden durch Benzyl, Fluor, Chlor, Brom oder durch geradkettiges oder verzweigtes Alkyl mit bis zu 3 Kohlenstoffatomen substituiert sein kann,
und deren Stereoisomere, Stereoisomerengemische und Salze.

4. Verbindungen der allgemeinen Formel (I) gemäß Anspruch 1, ausgewählt aus der Gruppe bestehend aus: 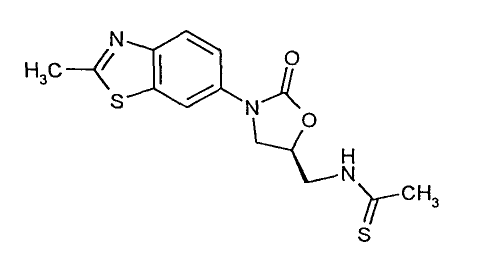 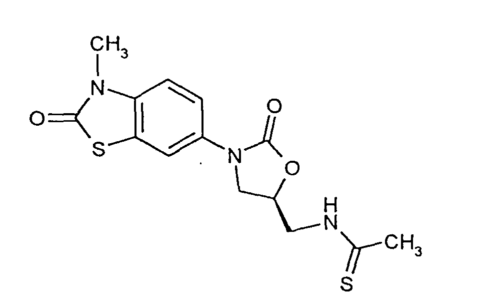 und

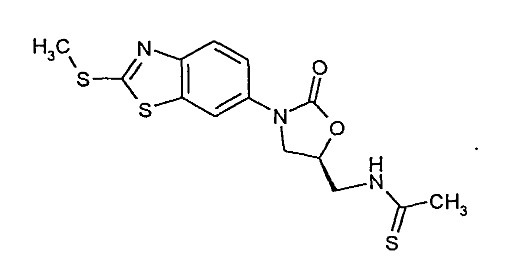

5. Verfahren zur Herstellung von Verbindungen der allgemeinen Formel (I) gemäß Anspruch 1, dadurch gekennzeichnet, daß man
[A] Verbindungen der allgemeinen Formel (II) 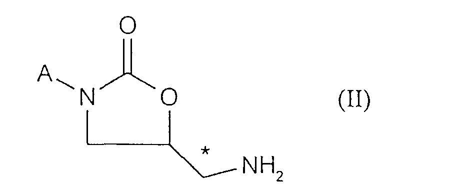 in welcher
A die in Anspruch 1 angegebene Bedeutung hat,
mit Verbindungen der allgemeinen Formel (III)
R¹-Y (III)
in welcher
R¹ die in Anspruch 1 angegebene Bedeutung hat,
und
Y in Abhängigkeit von R¹ für Wasserstoff, Halogen oder für C₁-C₄ geradkettiges oder verzweigtes Alkoxy, Oxyalkoxycarbonyl oder Pyridyl steht.
oder
[B] Verbindungen der allgemeinen Formel (IV) 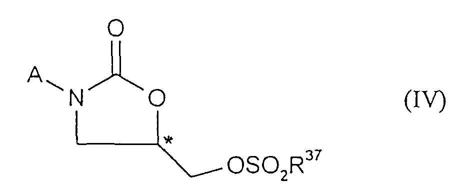 in welcher
A die oben angegebene Bedeutung hat,
R³⁷ für C₁-C₄-Alkyl steht,
mit Verbindungen der allgemeinen Formel (V)
NH₂-R^{1'} (V)
in welcher
R^{1'} für einen der oben unter R¹ aufgeführten Heterocyclen steht,
oder mit Ethyldithioacetat in inerten Lösemitteln, gegebenenfalls in Anwesenheit einer Base umsetzt,
im Fall der S-Oxide eine Oxidation nach üblicher Methode durchführt,
gegebenenfalls weitere Substituenten oder bereits vorhandene funktionelle Gruppen nach üblichen Methoden einführt bzw. derivatisiert,
gegebenenfalls die Verbindungen in bekannter Weise in die Salze überführt oder die Verbindungen aus ihren Salzen freisetzt,
und gegebenenfalls die Stereoisomere nach üblichen Methoden trennt.

6. Verbindungen der allgemeinen Formel (I) gemäß Anspruch 1 zur Verwendung bei der Bekämpfung von Krankheiten.

7. Verwendung von Verbindungen der allgemeinen Formel (I) gemäß Anspruch 1 zur Herstellung von Arzneimitteln.

8. Arzneimittel enthaltend Verbindungen der allgemeinen Formel (I) gemäß Anspruch 1.

## Claims

1. Substituted oxazolidinones of the general formula (I) 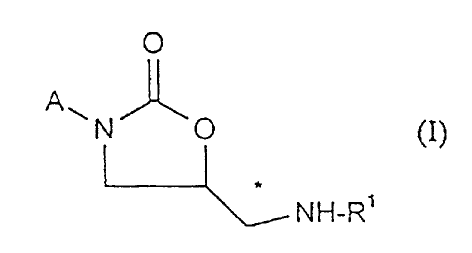 in which
A represents a 5-membered aromatic heterocycle bonded directly via a carbon atom and having up to 3 heteroatoms from the series S, N and/or O, which can additionally have a fused benzene or naphthyl ring, or
represents a 6-membered, aromatic heterocycle bonded directly via a carbon atom and having at least one nitrogen atom, or
represents an in each case 6-membered, bi- or tricyclic aromatic radical bonded directly via a carbon atom and having at least one nitrogen-containing ring, or
represents β-carbolin-3-yl or indolizinyl bonded directly via the 6-membered ring, the abovementioned cyclic systems optionally in each case being substituted up to 3 times in an identical or different manner by carboxyl, halogen, cyano, mercapto, formyl, phenyl, trifluoromethyl, nitro, straight-chain or branched alkoxy, alkoxycarbonyl, alkylthio or acyl each having up to 6 carbon atoms or by straight-chain or branched alkyl or alkenyl each having up to 6 carbon atoms, which for their part can be substituted by phenyl, and/or being substituted by pyridyl, which for its part can be substituted by straight-chain alkyl or alkoxy each having up to 6 carbon atoms or
represents a radical of the formula 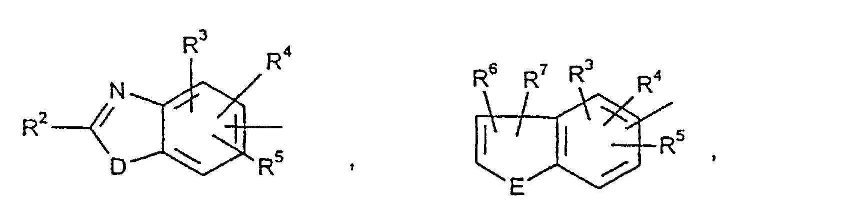 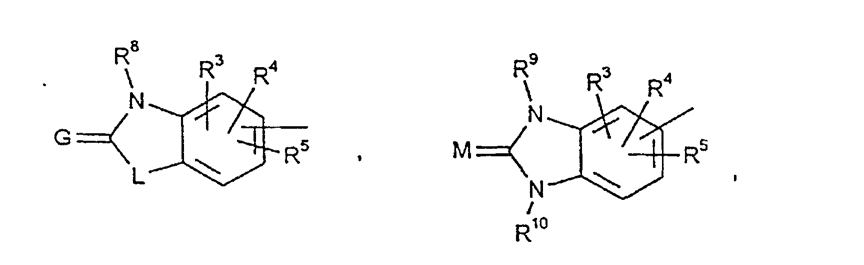 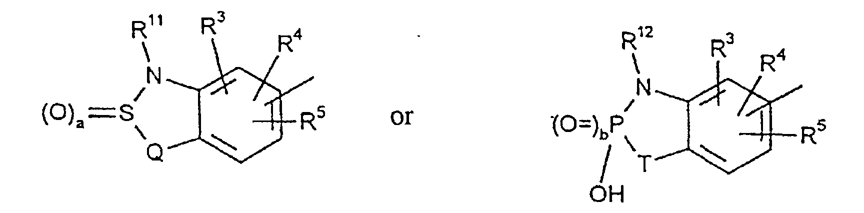 in which
R³, R⁴, R⁵, R⁶ and R⁷ are identical or different and denote hydrogen or carboxyl, halogen, cyano, formyl, trifluoromethyl, nitro, straight-chain or branched alkyl having up to 6 carbon atoms or a group of the formula -CO-NR¹³R¹⁴,
in which
R¹³ and R¹⁴ are identical or different and denote hydrogen, straight-chain or branched alkyl having up to 4 carbon atoms or phenyl,
R², R⁸, R⁹, R¹⁰, R¹¹ and R¹² are identical or different and denote hydrogen, cycloalkylcarbonyl or cycloalkyl each having 3 to 6 carbon atoms, or straight-chain or branched alkoxycarbonyl or alkylthio each having up to 6 carbon atoms, or denote straight-chain or branched alkyl having up to 10 carbon atoms, which is optionally substituted by cyano, trifluoromethyl, halogen, phenyl, hydroxyl, carboxyl, straight-chain or branched alkoxycarbonyl having up to 6 carbon atoms, aryl having 6 to 10 carbon atoms, cycloalkyl having 3 to 6 carbon atoms and/or by a group of the formula -(CO)_{c}-NR¹⁵R¹⁶, R¹⁷-N-SO₂-R¹⁸, R¹⁹R²⁰-N-SO₂- or R²¹-S(O)_{d},
in which
c denotes a number 0 or 1,
R¹⁵, R¹⁶ and R¹⁷ have the meaning of R¹³ and R¹⁴ indicated above and are identical to or different from this, or together with the nitrogen atom form a 5- to 6-membered, saturated heterocycle optionally having a further heteroatom from the series N, S and/or O, which for its part can be optionally substituted, also on a further nitrogen atom, by straight-chain or branched alkyl or acyl having up to 3 carbon atoms,
R¹⁹ and R²⁰ have the meaning of R¹³ and R¹⁴ indicated above and are identical to or different from this,
d denotes a number 0, 1 or 2,
R¹⁸ and R²¹ are identical or different and denote straight-chain or branched alkyl having up to 4 carbon atoms, benzyl, phenyl or tolyl,
or
denote straight-chain or branched acyl having up to 6 carbon atoms, which is optionally substituted by trifluoromethyl, trichloromethyl or by a group of the formula -OR²²,
in which
R²² denotes hydrogen or straight-chain or branched alkyl having up to 6 carbon atoms, which is optionally substituted by aryl having up to 10 carbon atoms,
or
denotes a group of the formula -(CO)ₑ-NR²³R²⁴, -NR²⁵-SO₂R²⁶, R²⁷R²⁸-NSO₂- or R²⁹-S(O)_{f},
in which
e has the meaning of c indicated above and is identical to or different from this,
R²³ and R²⁴ and R²⁵ each have the meaning of R¹⁵, R¹⁶ and R¹⁷ indicated above and are identical to or different from this,
R²⁷ and R²⁸ have the meaning of R¹³ and R¹⁴ indicated above and are identical to or different from this,
f has the meaning of d indicated above and is identical to or different from this,
R²⁶ and R²⁹ have the meaning of R¹⁸ and R²¹ in each case indicated above and are identical to or different from this,
D denotes an oxygen atom or a radical of the formula -S(O)_{g},
in which
g denotes a number 0, 1 or 2,
E and L are identical or different and denote an oxygen or a sulphur atom,
G, M, T and Q are identical or different and denote an oxygen or a sulphur atom, or a group of the formula -NR³⁰,
in which
R³⁰ denotes hydrogen or straight-chain or branched alkyl having up to 5 carbon atoms,
a and b are identical or different and denote a number 1 or 2,
R¹ represents a radical of the formula 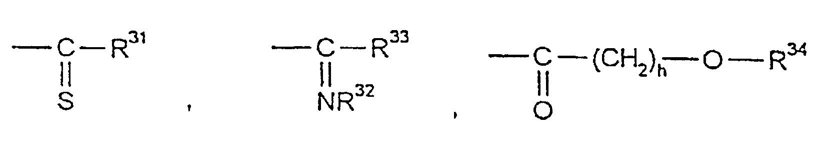 or 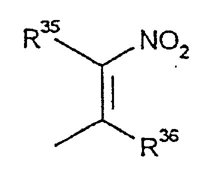 in which
R³¹ denotes straight-chain or branched alkyl having up to 7 carbon atoms, cycloalkyl having 3 to 6 carbon atoms, phenyl or a group of the formula -NR³⁸R³⁹,
in which
R³⁸ and R³⁹ have the meaning of R¹³ and R¹⁴ indicated above and are identical to or different from this,
R³² denotes hydrogen, cyano, cycloalkyl having 3 to 6 carbon atoms, phenyl or straight-chain or branched alkyl having up to 7 carbon atoms,
R³³ denotes hydrogen, straight-chain or branched alkyl having up to 7 carbon atoms, phenyl, cycloalkyl having 3 to 6 carbon atoms or a group of the formula -NR⁴⁰R⁴¹,
in which
R⁴⁰ and R⁴¹ have the meaning of R¹³ and R¹⁴ indicated above and are identical to or different from this,
h denotes a number 1, 2, 3 or 4,
R³⁴ denotes hydrogen, straight-chain or branched alkyl having up to 6 carbon atoms or benzyl,
R³⁵ and R³⁶ are identical or different and
denote hydrogen or straight-chain or branched alkyl having up to 6 carbon atoms,
or
R¹ represents cyano or a 5- to 7-membered, saturated, partially unsaturated or unsaturated heterocycle having up to 3 heteroatoms from the series S, N and/or O, which is optionally substituted, also via an N function, up to 2 times in an identical or different manner by benzyl, halogen or by straight-chain or branched alkyl having up to 5 carbon atoms,
and their stereoisomers, stereoisomer mixtures and salts.

2. Compounds of the general formula (I) according to Claim 1,
in which
A represents quinolyl, benzothiophenyl, benzothiazolyl, benzoxazolyl, pyridyl, pyridazyl or thienyl, each bonded via a carbon atom, each of which is optionally substituted up to 3 times in an identical or different manner by fluorine, chlorine, bromine, phenyl or by straight-chain or branched alkyl or alkylthio each having up to 4 carbon atoms or by straight-chain or branched alkenyl having up to 4 carbon atoms, which for its part can be substituted by phenyl, and/or is substituted by pyridyl, which for its part can be substituted by straight-chain or branched alkyl or alkoxy each having up to 5 carbon atoms, or represents a radical of the formula 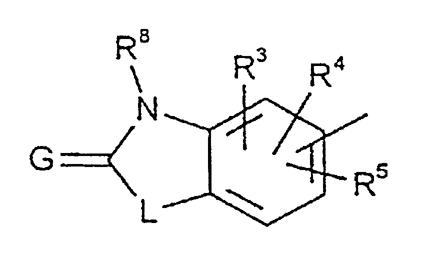 in which
G denotes an oxygen or sulphur atom,
L denotes an oxygen or sulphur atim,
R⁸ denotes straight-chain or branched alkyl or alkylthio each having up to 6 carbon atoms,
R³, R⁴ and R⁵ are identical or different and denote hydrogen, fluorine, chlorine or bromine,
R¹ represents a radical of the formula 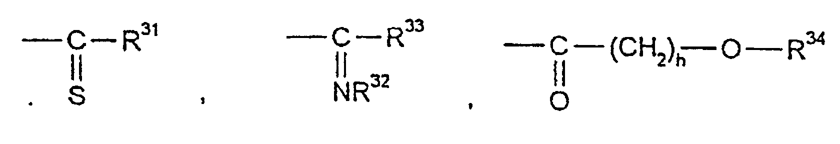 or 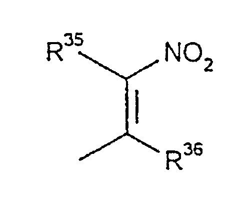 in which
R³¹ denotes straight-chain or branched alkyl having up to 5 carbon atoms, cyclopropyl, cyclobutyl, cyclopentyl, cyclohexyl, phenyl or a group of the formula -NR³⁸R³⁹,
in which
R³⁸ and R³⁹ are identical or different and denote hydrogen, methyl or ethyl,
R³² denotes hydrogen, cyano, cyclopropyl, cyclobutyl, cyclopentyl, cyclohexyl, phenyl or straight-chain or branched alkyl having up to 5 carbon atoms,
R³³ denotes hydrogen, straight-chain or branched alkyl having up to 5 carbon atoms, phenyl, cyclopropyl, cyclobutyl, cyclopentyl, cyclohexyl or a group of the formula -NR⁴⁰R⁴¹,
in which
R⁴⁰ and R⁴¹ have the meaning of R³⁸ and R³⁹ given above and are identical to or different from this,
h denotes a number 1, 2, 3 or 4,
R³⁴ denotes hydrogen, straight-chain or branched alkyl having up to 4 carbon atoms or benzyl,
R³⁵ and R³⁶ are identical or different and denote hydrogen or straight-chain or branched alkyl having up to 4 carbon atoms,
or
R¹ represents cyano or thienyl, oxazolyl, thiazolyl, isoxazolyl or pyrazolyl, each of which is optionally substituted, also via an N function, up to 2 times in an identical or different manner by benzyl, fluorine, chlorine, bromine or by straight-chain or branched alkyl having up to 3 carbon atoms,
and their stereoisomers, stereoisomer mixtures and salts.

3. Compounds of the general formula (I) according to Claim 1,
in which
A represents quinolyl, benzothiophenyl, benzothiazolyl, benzoxazolyl, pryidyl, pyridazyl or thienyl, each bonded via a carbon atom, each of which is optionally substituted up to 2 times in an identical or different manner by fluorine, chlorine, bromine, phenyl, by straight-chain or branched alkyl or alkylthio each having up to 3 carbon atoms or by straight-chain or branched alkenyl having up to 3 carbon atoms, which for its part can be substituted by phenyl, and/or is substituted by pyridyl, which for its part can be substituted by straight-chain or branched alkyl or alkoxy each having up to 4 carbon atoms, or
represents a radical of the formula 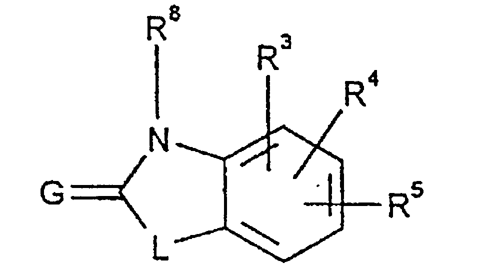 in which
G denotes an oxygen or sulphur atom,
L denotes an oxygen or sulphur atom,
R⁸ denotes straight-chain or branched alkyl having up to 4 carbon atoms,
R³, R⁴ and R⁵ are identical or different and
denote hydrogen, fluorine, chlorine or bromine,
R¹ represents a radical of the formula 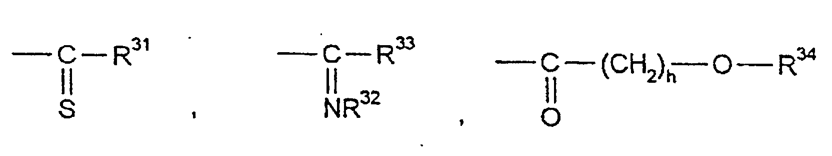 or 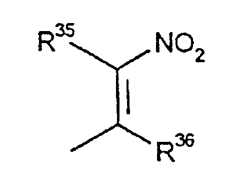 in which
R³¹ denotes straight-chain or branched alkyl having up to 4 carbon atoms, cyclopropyl, cyclobutyl, cyclopentyl, cyclohexyl, phenyl or a group of the formula -NR³⁸R³⁹,
in which
R³⁸ and R³⁹ are identical or different and denote hydrogen or methyl,
R³² denotes hydrogen, cyano, cyclopropyl, cyclobutyl, cyclopentyl, cyclohexyl, phenyl or straight-chain or branched alkyl having up to 4 carbon atoms,
R³³ denotes hydrogen, straight-chain or branched alkyl having up to 4 carbon atoms, phenyl, cyclopropyl, cyclobutyl, cyclopentyl, cyclohexyl or a group of the formula -NR⁴⁰R⁴¹,
in which
R⁴⁰ and R⁴¹ have the meaning of R³⁸ and R³⁹ given above and are identical to or different from this,
h denotes a number 1, 2, 3 or 4,
R³⁴ denotes hydrogen, straight-chain or branched alkyl having up to 3 carbon atoms or benzyl,
R³⁵ and R³⁶ are identical or different and
denote hydrogen or straight-chain or branched alkyl having up to 3 carbon atoms,
or
R¹ represents cyano or thienyl, thiazolyl, isoxazolyl or pyrazolyl, each of which can optionally be substituted, also via an N function, up to 2 times in an identical or different manner by benzyl, fluorine, chlorine, bromine or by straight-chain or branched alkyl having up to 3 carbon atoms,
and their stereoisomers, stereoisomer mixtures and salts.

4. Compounds of the general formula (I) according to Claim 1, selected from the group consisting of: 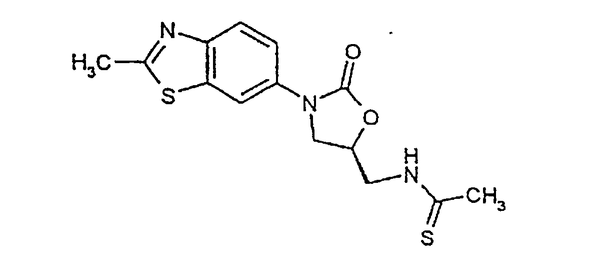 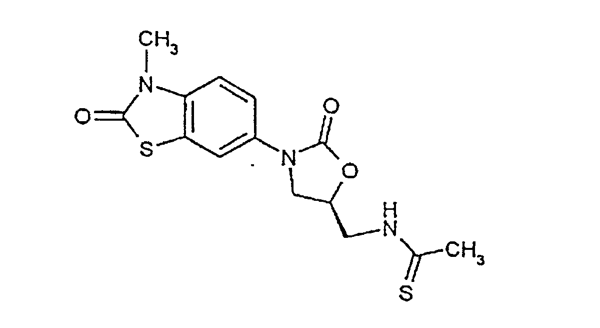 and

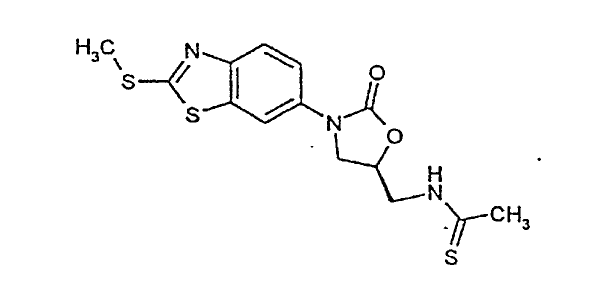

5. Process for the preparation of compounds of the general formula (I) according to Claim 1, characterized in that
[A] compounds of the general formula (II) 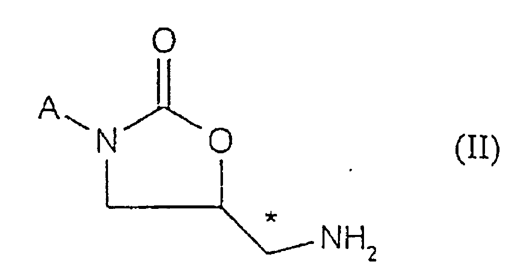 in which
A has the meaning indicated in Claim 1,
are reacted with compounds of the general formula (III)
R¹-Y (III)
in which
R¹ has the meaning indicated in Claim 1,
and
Y, depending on R¹, represents hydrogen, halogen or C₁-C₄ straight-chain or branched alkoxy, oxyalkoxycarbonyl or pyridyl,
or
[B] compounds of the general formula (IV) 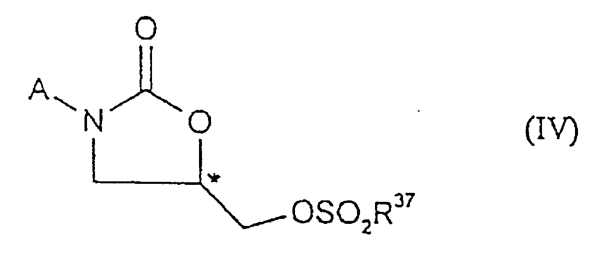 in which
A has the meaning indicated above,
R³⁷ represents C₁-C₄-alkyl,
are reacted with compounds of the general formula (V)
NH₂-R^{1'} (V)
in which
R^{1'} represents one of the heterocycles listed above under R¹,
or with ethyl dithioacetate in inert solvents, if appropriate in the presence of a base,
and in the case of the S-oxides an oxidation is carried out according to a customary method,
and, if appropriate, further substituents or functional groups already present are introduced or derivatized according to customary methods,
if appropriate the compounds are converted into the salts in a known manner or the compounds are liberated from their salts,
and if appropriate the stereoisomers are separated according to customary methods.

6. Compounds of the general formula (I) according to Claim 1 for use in the control of diseases.

7. Use of compounds of the general formula (I) according to Claim 1 for the production of medicaments.

8. Medicaments comprising compounds of the general formula (1) according to Claim 1.

## Revendications

1. Oxazolidinones substituées répondant à la formule générale (I) 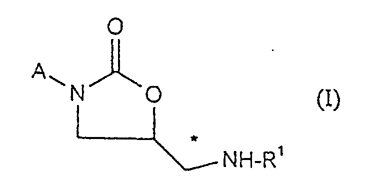 dans laquelle
A représente un radical hétérocyclique aromatique pentagonal lié de manière directe via un atome de carbone, contenant jusqu'à 3 hétéroatomes choisis parmi la série comprenant un atome de soufre, un atome d'azote et/ou un atome d'oxygène, qui peut en outre posséder un noyau naphtyle ou benzénique condensé, ou
représente un radical hétérocyclique aromatique hexagonal lié de manière directe via un atome de carbone, contenant au moins un atome d'azote, ou
représente un radical aromatique respectivement hexagonal, bicyclique ou tricyclique, lié de manière directe via un atome de carbone, comprenant au moins un noyau azoté, ou
représente un groupe β-carbolin-3-yle ou représente un groupe indolizinyle lié de manière directe via le noyau hexagonal, les cycles mentionnés ci-dessus pouvant, le cas échéant, porter respectivement jusqu'à 3 substituants identiques ou différents carboxyle, halogéno, cyano, mercapto, formyle, phényle, trifluorométhyle, nitro, alcoxy à chaîne droite ou ramifiée, alcoxycarbonyle, alkylthio ou acyle contenant respectivement jusqu'à 6 atomes de carbone, ou jusqu'à 3 substituants alkyle ou alcényle à chaîne droite ou ramifiée contenant respectivement jusqu'à 6 atomes de carbone, ces derniers substituants peuvant porter, quant à eux, un ou plusieurs substituants phényle, et/ou jusqu'à 3 substituants pyridyle, ce dernier substituant pouvant porter, quant à lui, un ou plusieurs substituants alkyle ou alcoxy à chaîne droite contenant respectivement jusqu'à 6 atomes de carbone, ou
représente un radical répondant aux formules 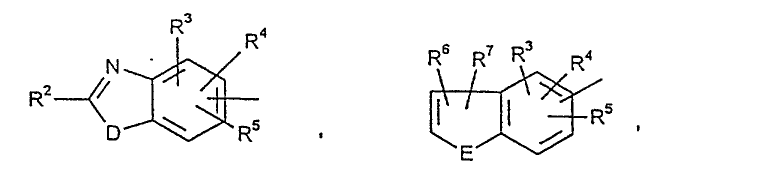 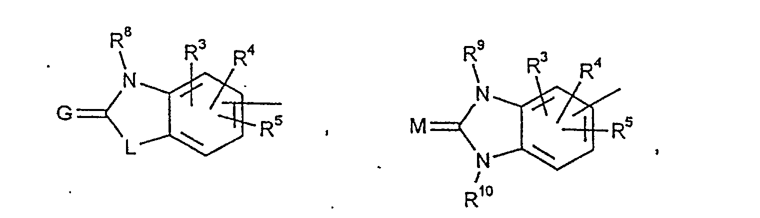 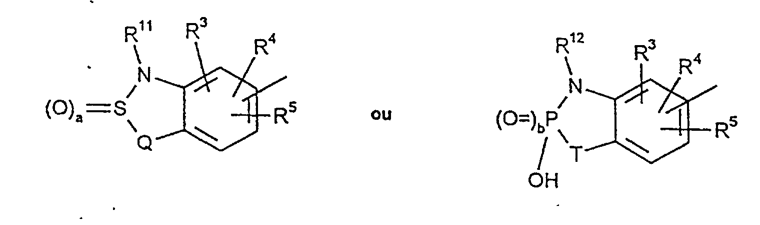 dans lesquelles
R³, R⁴, R⁵, R⁶ et R⁷ sont identiques ou différents et représentent un atome d'hydrogène ou un groupe carboxyle, un atome d'halogène, un groupe cyano, un groupe formyle, un groupe trifluorométhyle, un groupe nitro, un groupe alkyle à chaîne droite ou ramifiée contenant jusqu'à 6 atomes de carbone ou représentent un groupe répondant à la formule -CO-NR¹³R¹⁴,
dans laquelle
R¹³ et R¹⁴ sont identiques ou différents et représentent un atome d'hydrogène, un groupe alkyle à chaîne droite ou ramifiée contenant jusqu'à 4 atomes de carbone ou encore un groupe phényle,
R², R⁸, R⁹, R¹⁰, R¹¹ et R¹² sont identiques ou différents et représentent un atome d'hydrogène, un groupe cycloalkylcarbonyle ou un groupe cycloalkyle contenant respectivement de 3 à 6 atomes de carbone, ou représentent un groupe alcoxycarbonyle ou un groupe alkylthio à chaîne droite ou ramifiée contenant respectivement jusqu'à 6 atomes de carbone, ou représentent un groupe alkyle à chaîne droite ou ramifiée contenant jusqu'à 10 atomes de carbone, qui porte, le cas échéant, un ou plusieurs substituants cyano, trifluorométhyle, halogéno, phényle, hydroxyle, carboxyle, alcoxycarbonyle à chaîne droite ou ramifiée contenant jusqu'à 6 atomes de carbone, aryle contenant de 6 à 10 atomes de carbone, cycloalkyle contenant de 3 à 6 atomes de carbone et/ou, à titre de substituant, un groupe répondant aux formules -(CO)_{c}-NR¹⁵R¹⁶, R¹⁷-N-SO₂-R¹⁸, R¹⁹R²⁰-NSO₂- ou -R²¹-S(O)_{d},
dans lesquelles
c représente le nombre 0 ou 1,
R¹⁵, R¹⁶ et R¹⁷ ont la signification de R¹³ et de R¹⁴ indiquée ci-dessus et sont identiques ou différents par rapport à ces derniers,
ou forment ensemble avec l'atome d'azote un groupe hétérocyclique saturé penta- à hexagonal contenant,
le cas échéant, un hétéro-atome supplémentaire choisi parmi la série comprenant un atome d'azote, un atome de soufre et/ou un. atome d'oxygène, ce groupe pouvant, quant à lui, porter, sur un autre atome d'azote, un ou plusieurs substituants alkyle ou acyle à chaîne droite ou ramifiée contenant jusqu'à 3 atomes de carbone,
R¹⁹ et R²⁰ ont la signification de R¹³ et de R¹⁴ indiquée ci-dessus et sont identiques ou différents par rapport à ces derniers,
d représente le nombre 0, 1 ou 2,
R¹⁸ et R²¹ sont identiques ou différents et représentent un groupe alkyle à chaîne droite ou ramifiée contenant jusqu'à 4 atomes de carbone, un groupe benzyle, un groupe phényle ou un groupe tolyle,
ou
représentent un groupe acyle à chaîne droite ou ramifiée contenant jusqu'à 6 atomes de carbone qui porte, le cas échéant, un ou plusieurs substituants trifluorométhyle, trichlorométhyle ou encore, à titre de substituant, un groupe répondant à la formule -OR²²,
dans laquelle
R²² représente un atome d'hydrogène ou un groupe alkyle à chaîne droite ou ramifiée contenant jusqu'à 6 atomes de carbone, qui porte, le cas échéant, un ou plusieurs substituants aryle contenant jusqu'à 10 atomes de carbone,
ou
représentent un groupe répondant aux formules -(CO)ₑ-NR²³R²⁴, NR²⁵-SO₂-R²⁶, R²⁷R²⁸-NSO₂- ou -R²⁹-S(O)_{f},
dans lesquelles
e a la signification de c indiquée ci-dessus et est identique ou différent par rapport à ce dernier,
R²³, R²⁴ et R²⁵ ont respectivement la signification de R¹⁵, de R¹⁶ et de R¹⁷ indiquée ci-dessus et sont identiques ou différents par rapport à ces derniers,
R²⁷ et R²⁸ ont la signification de R¹³ et de R¹⁴ indiquée ci-dessus et sont identiques ou différents par rapport à ces derniers,
f a la signification de d indiquée ci-dessus et est identique ou différent par rapport à ce dernier,
R²⁶ et R²⁹ ont la signification de.R¹⁸ et de R²¹ respectivement indiquée ci-dessus et sont identiques ou différents par rapport à ces derniers,
D représente un atome d'oxygène ou un radical répondant à la formule -S(O)_{g},
dans laquelle
g représente le nombre 0, 1 ou 2,
E et L sont identiques ou différents et représentent un atome d'oxygène ou un atome de soufre,
G, M, T et Q sont identiques ou différents et représentent un atome d'oxygène ou un atome de soufre ou encore un groupe répondant à la formule -NR³⁰,
dans laquelle
R³⁰ représente un atome d'hydrogène ou un groupe alkyle à chaîne droite ou ramifiée contenant jusqu'à 5 atomes de carbone,
a et b sont identiques ou différents et représentent le nombre 1 ou 2,
R¹ représente un radical répondant aux formules 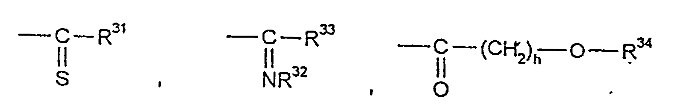 ou 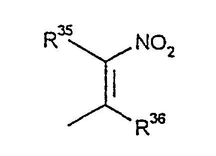 dans lesquelles
R³¹ représente un groupe alkyle à chaîne droite ou ramifiée contenant jusqu'à 7 atomes de carbone, un groupe cycloalkyle contenant de 3 à 6 atomes de carbone, un groupe phényle ou représente un groupe répondant à la formule -NR³⁸R³⁹,
dans laquelle
R³⁸ et R³⁹ ont la signification de R¹³ et de R¹⁴ indiquée ci-dessus et sont identiques ou différents par rapport à ces derniers,
R³² représente un atome d'hydrogène, un groupe cyano, un groupe cycloalkyle contenant de 3 à 6 atomes de carbone, un groupe phényle ou représente un groupe alkyle à chaîne droite ou ramifiée contenant jusqu'à 7 atomes de carbone,
R³³ représente un atome d'hydrogène, un groupe alkyle à chaîne droite ou ramifiée contenant jusqu'à 7 atomes de carbone, un groupe phényle, un groupe cycloalkyle contenant de 3 à 6 atomes de carbone ou représente un groupe répondant à la formule -NR⁴⁰R⁴¹,
dans laquelle
R⁴⁰ et R⁴¹ ont la signification de R¹³ et de R¹⁴ indiquée ci-dessus et sont identiques ou différents par rapport à ces derniers,
h représente le nombre 1, 2, 3 ou 4,
R³⁴ représente un atome d'hydrogène, un groupe alkyle à chaîne droite ou ramifiée contenant jusqu'à 6 atomes de carbone ou un groupe benzyle,
R³⁵ et R³⁶ sont identiques ou différents et représentent un atome d'hydrogène ou un groupe alkyle à chaîne droite ou ramifiée contenant jusqu'à 6 atomes de carbone,
ou bien
R¹ représente un groupe cyano ou un groupe hétérocyclique penta- à heptagonal, saturé, partiellement non saturé ou non saturé contenant jusqu'à 3 hétéroatomes choisis parmi la série comprenant un atome de soufre, un atome d'azote et/ou un atome d'oxygène, qui, le cas échéant, portent également, via une fonction azote, jusqu'à 2 substituants identiques ou différents benzyle, halogéno ou qui portent jusqu'à 2 substituants alkyle contenant jusqu'à 5 atomes de carbone,
ainsi que leurs stéréo-isomères, leurs mélanges de stéréo-isomères et leurs sels.

2. Composés répondant à la formule générale (I) selon la revendication 1,
dans laquelle
A représente un groupe quinolyle, un groupe benzothiophényle, un groupe benzothiazolyle, un groupe benzoxazolyle, un groupe pyridyle, un groupe pyridazyle ou un groupe thiényle, liés respectivement via un atome de carbone, qui portent, le cas échéant, jusqu'à 3 substituants identiques ou différents fluoro, chloro, bromo, phényle, ou jusqu'à 3 substituants alkyle ou alkylthio à chaîne droite ou ramifiée contenant respectivement jusqu'à 4 atomes de carbone, ou jusqu'à 3 substituants alcényle à chaîne droite ou ramifiée contenant jusqu'à 4 atomes de carbone, ce dernier substituant pouvant, quant à lui, porter un ou plusieurs substituants phényle, et/ou jusqu'à 3 substituants pyridyle qui, quant à lui, peut porter un ou plusieurs substituants alkyle ou alcoxy à chaîne droite ou ramifiée contenant respectivement jusqu'à 5 atomes de carbone, ou
représente un radical répondant à la formule 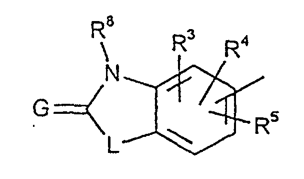 dans laquelle
G représente un atome d'oxygène ou un atome de soufre,
L représente un atome d'oxygène ou un atome de soufre,
R⁸ représente un groupe alkyle ou un groupe alkylthio à chaîne droite ou ramifiée contenant respectivement jusqu'à 6 atomes de carbone,
R³, R⁴ et R⁵ sont identiques ou différents et représentent un atome d'hydrogène, un atome de fluor, un atome de chlore ou un atome de brome,
R¹ représente un radical répondant aux formules 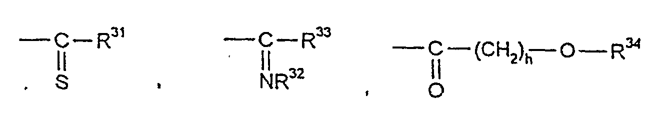 ou 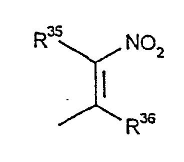 dans lesquelles
R³¹ représente un groupe alkyle à chaîne droite ou ramifiée contenant jusqu'à 5 atomes de carbone, un groupe cyclopropyle, un groupe cyclobutyle, un groupe cyclopentyle, un groupe cyclohexyle, un groupe phényle ou représente un groupe répondant à la formule -NR³⁸R³⁹,
dans laquelle
R³⁸ et R³⁹ sont identiques ou différents et représentent un atome d'hydrogène, un groupe méthyle ou un groupe éthyle,
R³² représente un atome d'hydrogène, un groupe cyano, un groupe cyclopropyle, un groupe cyclobutyle, un groupe cyclopentyle, un groupe cyclohexyle, un groupe phényle ou représente un groupe alkyle à chaîne droite ou ramifiée contenant jusqu'à 5 atomes de carbone,
R³³ représente un atome d'hydrogène, un groupe alkyle à chaîne droite ou ramifiée contenant jusqu'à 5 atomes de carbone, un groupe phényle, un groupe cyclopropyle,
un groupe cyclobutyle, un groupe cyclopentyle, un groupe cyclohexyle ou représente un groupe répondant à la formule -NR⁴⁰R⁴¹,
dans laquelle
R⁴⁰ et R⁴¹ ont la signification de R³⁸ et de R³⁹ indiquée ci-dessus et sont identiques ou différents par rapport à ces derniers,
h représente le nombre 1, 2, 3 ou 4,
R³⁴ représente un atome d'hydrogène, un groupe alkyle à chaîne droite ou ramifiée contenant jusqu'à 4 atomes de carbone ou un groupe benzyle,
R³⁵ et R³⁶ sont identiques ou différents et représentent un atome d'hydrogène ou un groupe alkyle à chaîne droite ou ramifiée contenant jusqu'à 4 atomes de carbone,
ou bien
R¹ représente un groupe cyano ou représente un groupe thiényle, un groupe oxazolyle, un groupe thiazolyle, un groupe isoxazolyle ou un groupe pyrazolyle, ces derniers groupes portant également, le cas échéant, via une fonction azote, jusqu'à 2 substituants identiques ou différents benzyle, fluoro, chloro, bromo ou jusqu'à 2 substituants alkyle contenant jusqu'à 3 atomes de carbone,
ainsi que leurs stéréo-isomères, leurs mélanges de stéréo-isomères et leurs sels.

3. Composés répondant à la formule générale (I) selon la revendication 1,
dans laquelle
A représente un groupe quinolyle, un groupe benzothiophényle, un groupe benzothiazolyle, un groupe benzoxazolyle, un groupe pyridyle, un groupe pyridazyle ou un groupe thiényle, liés respectivement via un atome de carbone, qui portent, le cas échéant, jusqu'à 2 substituants identiques ou différents fluoro, chloro, bromo, phényle, ou jusqu'à 2 substituants alkyle ou alkylthio à chaîne droite ou ramifiée contenant respectivement jusqu'à 3 atomes de carbone, ou jusqu'à 2 substituants alcényle à chaîne droite ou ramifiée contenant jusqu'à 3 atomes de carbone, ce dernier substituant pouvant, quant à lui, porter un ou plusieurs substituants phényle, et/ou jusqu'à 2 substituants pyridyle qui, quant à lui, peut porter un ou plusieurs substituants alkyle ou alcoxy à chaîne droite ou ramifiée contenant respectivement jusqu'à 4 atomes de carbone, ou
représente un radical répondant à la formule 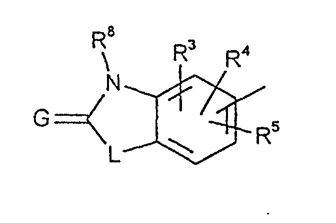 dans laquelle
G représente un atome d'oxygène ou un atome de soufre,
L représente un atome d'oxygène ou un atome de soufre,
R⁸ représente un groupe alkyle à chaîne droite ou ramifiée contenant jusqu'à 4 atomes de carbone,
R³, R⁴ et R⁵ sont identiques ou différents et représentent un atome d'hydrogène, un atome de fluor, un atome de chlore ou un atome de brome,
R¹ représente un radical répondant aux formules 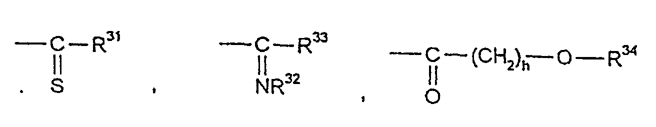 ou 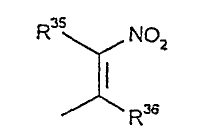 dans lesquelles
R³¹ représente un groupe alkyle à chaîne droite ou ramifiée contenant jusqu'à 4 atomes de carbone, un groupe cyclopropyle, un groupe cyclobutyle, un groupe cyclopentyle, un groupe cyclohexyle, un groupe phényle ou représente un groupe répondant à la formule -NR³⁸R³⁹,
dans laquelle
R³⁸ et R³⁹ sont identiques ou différents et représentent un atome d'hydrogène ou un groupe méthyle,
R³² représente un atome d'hydrogène, un groupe cyano, un groupe cyclopropyle, un groupe cyclobutyle, un groupe cyclopentyle, un groupe cyclohexyle, un groupe phényle ou représente un groupe alkyle à chaîne droite ou ramifiée contenant jusqu'à 4 atomes de carbone,
R³³ représente un atome d'hydrogène, un groupe alkyle à chaîne droite ou ramifiée contenant jusqu'à 4 atomes de carbone, un groupe phényle, un groupe cyclopropyle, un groupe cyclobutyle, un groupe cyclopentyle, un groupe cyclohexyle ou représente un groupe répondant à la formule -NR⁴⁰R⁴¹,
dans laquelle
R⁴⁰ et R⁴¹ ont la signification de R³⁸ et de R³⁹ indiquée ci-dessus et sont identiques ou différents par rapport à ces derniers,
h représente le nombre 1, 2, 3 ou 4,
R³⁴ représente un atome d'hydrogène, un groupe alkyle à chaîne droite ou ramifiée contenant jusqu'à 3 atomes de carbone ou un groupe benzyle,
R³⁵ et R³⁶ sont identiques ou différents et représentent un atome d'hydrogène ou un groupe alkyle à chaîne droite ou ramifiée contenant jusqu'à 3 atomes de carbone,
ou bien
R¹ représente un groupe cyano ou représente un groupe thiényle, un groupe thiazolyle, un groupe isoxazolyle ou un groupe pyrazolyle, ces derniers groupes pouvant également porter, le cas échéant, via une fonction azote, jusqu'à 2 substituants identiques ou différents benzyle, fluoro, chloro, bromo ou jusqu'à 2 substituants alkyle contenant jusqu'à 3 atomes de carbone,
ainsi que leurs stéréo-isomères, leurs mélanges de stéréo-isomères et leurs sels.

4. Composés répondant à la formule générale (I) selon la revendication 1, choisis parmi le groupe constitué par: 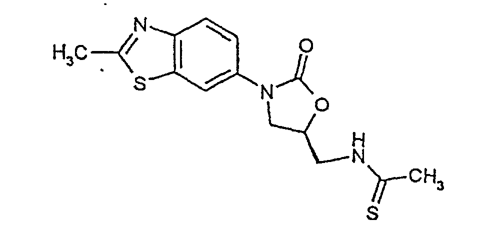 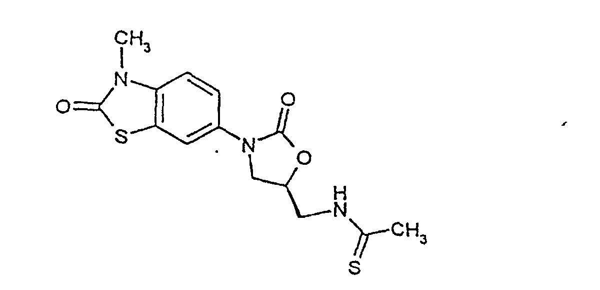 et

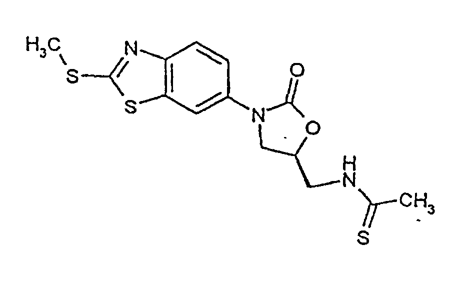

5. Procédé pour la préparation de composés répondant à la formule générale (I) selon la revendication 1, caractérisé en ce qu'on fait réagir:
[A] des composés répondant à la formule générale (II): 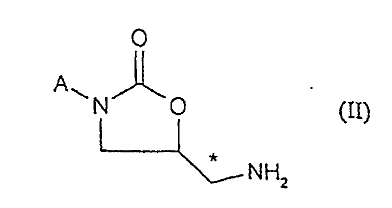 dans laquelle
A a la signification indiquée à la revendication 1,
avec des composés répondant à la formule générale (III)
R¹-Y (III)
dans laquelle
R¹ a la signification indiquée à la revendication 1,
et
Y représente, en fonction de R¹, un atome d'hydrogène, un atome d'halogène ou représente un groupe alcoxy en C₁-C₄ à chaîne droite ou ramifiée, un groupe oxyalcoxycarbonyle ou un groupe pyridyle,
ou
[B] des composés répondant à la formule générale (IV): 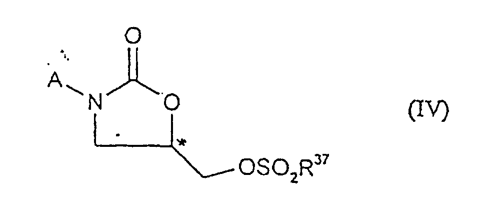 dans laquelle
A a la signification indiquée ci-dessus,
R³⁷ représente un groupe alkyle en C₁-C₄,
avec des composés répondant à la formule générale (V)
NH₂-R^{1'} (V)
dans laquelle
R¹' représente un des groupes hétérocycliques indiqués ci-dessus sous R¹',
ou avec de l'éthyldithioacétate dans des solvants inertes, le cas échéant, en présence d'une base,
dans le cas des oxydes de soufre, on procède à une oxydation conformément aux procédés habituels,
le cas échéant, on introduit, respectivement on dérive des substituants supplémentaires ou des groupes fonctionnels déjà présents, conformément aux procédés habituels,
le cas échéant, on transforme les composés de manière connue en sels ou bien on libère les composés de leurs sels,
et, le cas échéant, on sépare les stéréo-isomères conformément aux procédés habituels.

6. Composés répondant à la formule générale (I) selon la revendication 1 à utiliser dans la lutte contre des maladies.

7. Utilisation des composés répondant à la formule générale (I) selon la revendication 1 pour la préparation de médicaments.

8. Médicaments contenant des composés répondant à la formule générale (I) selon la revendication 1.
